# EUROPÄISCHE PATENTSCHRIFT

(11) **EP 3 160 954 B1**
(45) Veröffentlichungstag und Bekanntmachung des Hinweises auf die Patenterteilung: **23.09.2020**
(21) Anmeldenummer: 15734292.4
(22) Anmeldetag: 29.05.2015
(51) Int. Cl.: C07D 311/82, C07D 311/96, C07D 487/10, H01L 51/05, H01L 51/42, H01L 51/50

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 25.06.2014 EP 14002178
(43) Veröffentlichungstag der Anmeldung: 03.05.2017
(73) Patentinhaber: Merck Patent GmbH, 64293 Darmstadt (DE)
(72) Erfinder: PARHAM, Amir Hossain, 60486 Frankfurt am Main (DE); STOESSEL, Philipp, 60389 Frankfurt am Main (DE); EBERLE, Thomas, 76829 Landau (DE); JATSCH, Anja, 60489 Frankfurt am Main (DE); KROEBER, Jonas Valentin, 60311 Frankfurt am Main (DE); GROSSMANN, Tobias, 64297 Darmstadt (DE); PFLUMM, Christof, 64291 Darmstadt (DE)
(86) Internationale Anmeldenummer: PCT/EP2015/001096
(87) Internationale Veröffentlichungsnummer: WO 2015/197156

(56) Entgegenhaltungen:
- WO-A1-2013/149958
- WO-A1-2014/072107
- CN-A- 102 786 508
- JP-A- 2010 202 599

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen enthaltend diese Materialien.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei zunehmend metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, jedoch immer noch Verbesserungsbedarf, beispielsweise im Hinblick auf Effizienz, Betriebsspannung und Lebensdauer.

Die Eigenschaften phosphoreszierender OLEDs werden nicht nur von den eingesetzten Triplettemittern bestimmt. Hier sind insbesondere auch die anderen verwendeten Materialien, wie Matrixmaterialien, von besonderer Bedeutung. Verbesserungen dieser Materialien und deren Ladungstransporteigenschaften können somit auch zu deutlichen Verbesserungen der OLED-Eigenschaften führen.

Gemäß dem Stand der Technik werden unter anderem Indolocarbazolderivate, z. B. gemäß WO 2007/063754 oder WO 2008/056746, Indenocarbazolderivate, z. B. gemäß WO 2010/136109, oder Fluoren- bzw. Spirobifluorenderivate, z. B. gemäß WO 2012/074210, als Matrixmaterialien für phosphoreszierende Emitter in organischen Elektrolumineszenzvorrichtungen eingesetzt. Hier sind weitere Verbesserungen wünschenswert, insbesondere in Bezug auf die Effizienz, die Lebensdauer und die Betriebsspannung.

Aufgabe der vorliegenden Erfindung ist die Bereitstellung von Verbindungen, welche sich für den Einsatz in einer OLED eignen, insbesondere als Matrixmaterial für phosphoreszierende Emitter. Eine weitere Aufgabe der vorliegenden Erfindung ist es, weitere organische Halbleiter für organische Elektrolumineszenzvorrichtungen bereitzustellen, um so dem Fachmann eine größere Wahlmöglichkeit an Materialien für die Herstellung von OLEDs zu ermöglichen.

Überraschend wurde gefunden, dass bestimmte, unten näher beschriebene Verbindungen diese Aufgabe lösen, sich gut für die Verwendung in OLEDs eignen und zu Verbesserungen der organischen Elektrolumineszenzvorrichtung führen. Dabei betreffen die Verbesserungen insbesondere die Lebensdauer, die Effizienz und/oder die Betriebsspannung. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Aus der WO 2013/017189 sind Spirobifluorenderivate mit ankondensierter Carbazoleinheit bekannt. Verbindungen gemäß der vorliegenden Erfindung werden nicht offenbart, und es wird nicht offenbart, dass Verbindungen, dass Verbindungen, die eine Sauerstoff- oder Schwefelbrücke aufweisen, zu technischen Vorteilen führen. Aus WO 2013/149958 und CN 102786508 sind Aryl- bzw. Heteroaryl-substituierte Spiroxanthenverbindungen für die Verwendung in OLEDs bekannt. Aus JP 2010-202599 sind Spiroxanthenverbindungen mit ankondensierten Xanthengruppen bekannt. Verbindungen mit ankondensierter Indoleinheit sind nicht offenbart.

Gegenstand der vorliegenden Erfindung ist daher eine Verbindung gemäß Formel (1), 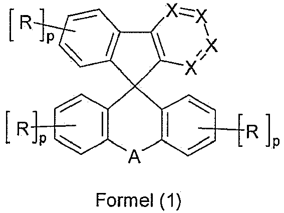 wobei für die verwendeten Symbole und Indizes gilt:
- A: ist O oder S;
- X: zwei benachbarte X stehen für eine Gruppe der folgenden Formel (2), 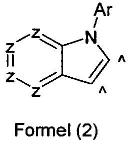 wobei ^ die entsprechenden benachbarten Gruppen X in Formel (1) andeutet; und die verbleibenden beiden Gruppen X stehen für CR;
- Z: steht für CR; oder zwei benachbarte Z stehen für eine Gruppe der folgenden Formel (2a) und die anderen beiden Z stehen für CR, 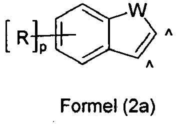 wobei ^ die entsprechenden benachbarten Gruppen Z in Formel (2) andeutet; dabei steht W für O, S, NR oder CR₂;
- Ar: ist ein aromatisches oder heteroaromatisches Ringsystem mit 5 bis 40 aromatischen Ringatomen, das mit einem oder mehreren Resten R substituiert sein kann;
- R: ist bei jedem Auftreten gleich oder verschieden ausgewählt aus der Gruppe bestehend aus H, D, F, Cl, Br, I, CN, NO₂, N(Ar¹)₂, N(R¹)₂, C(=O)Ar¹, C(=O)R¹, P(=O)(Ar¹)₂, P(Ar¹)₂, B(Ar¹)₂, Si(Ar¹)₃, Si(R¹)₃, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R¹ substituiert sein kann, wobei eine oder mehrere nicht-benachbarte CH₂-Gruppen durch R¹C=CR¹, C≡C, Si(R¹)₂, C=O, C=S, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R¹ substituiert sein kann, einer Aryloxy- oder Heteroaryloxygruppe mit 5 bis 40 aromatischen Ringatomen, die mit einem oder mehreren Resten R¹ substituiert sein kann; dabei können optional zwei benachbarte Substituenten R ein monocyclisches oder polycyclisches, aliphatisches, aromatisches oder heteroaromatisches Ringsystem bilden, das mit einem oder mehreren Resten R¹ substituiert sein kann;
- Ar¹: ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R¹ substituiert sein kann; dabei können zwei Reste Ar¹, welche an dasselbe N-, P-, B- oder Si-Atom binden, auch durch eine Einfachbindung oder eine Brücke, ausgewählt aus N(R¹), C(R¹)₂, O oder S, miteinander verbrückt sein;
- R¹: ist bei jedem Auftreten gleich oder verschieden ausgewählt aus der Gruppe bestehend aus H, D, F, CN, einem aliphatischen Kohlenwasserstoffrest mit 1 bis 20 C-Atomen, oder einem aromatischen oder heteroaromatischen Ringsystem mit 5 bis 30 aromatischen Ringatomen, in dem ein oder mehrere H-Atome durch D, F, CN oder eine Alkylgruppe mit 1 bis 10 C-Atomen ersetzt sein können; dabei können wobei zwei oder mehr benachbarte Substituenten R¹ miteinander ein mono- oder polycyclisches, aliphatisches Ringsystem bilden;
- p: ist bei jedem Auftreten gleich oder verschieden 0, 1, 2, 3 oder 4.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

Unter benachbarten Gruppen X bzw. Z im Sinne dieser Anmeldung werden Gruppen X bzw. Z verstanden, die direkt aneinander gebunden sind.

Unter benachbarten Resten bzw. benachbarten Substituenten im Sinne der vorliegenden Anmeldung werden Substituenten verstanden, die an C-Atome gebunden sind, welche wiederum direkt aneinander gebunden sind, oder Substituenten, die an dasselbe C-, Si-, N-, P- oder B-Atom gebunden sind.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

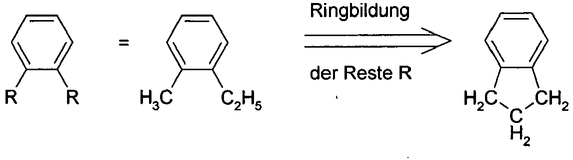

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Die Gruppe der Formel (2) kann in verschiedenen Positionen innerhalb der Verbindung der Formel (1) gebunden sein. Ausführungsformen der vorliegenden Erfindung sind daher die Verbindungen der folgenden Formeln (3) bis (8), 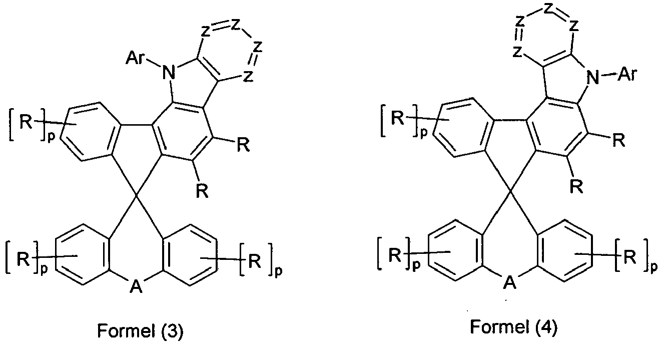 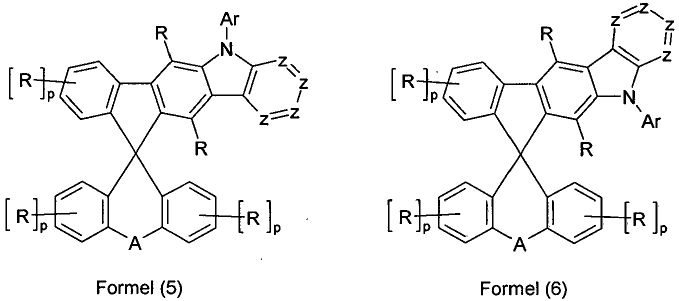 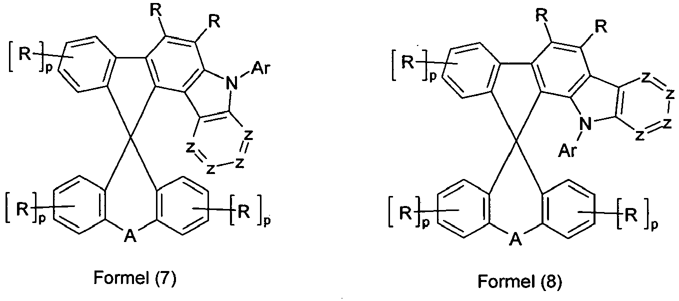 wobei die verwendeten Symbole und Indizes die oben genannten Bedeutungen aufweisen.

In einer bevorzugten Ausführungsform der Formeln (1) bis (8) ist p bei jedem Auftreten gleich oder verschieden 0, 1, 2 oder 3, besonders bevorzugt 0, 1 oder 2 und ganz besonders bevorzugt gleich 0 oder 1.

Bevorzugte Ausführungsformen der Strukturen gemäß Formel (3) bis (8) sind die Strukturen der Formeln (3a) bis (8a), 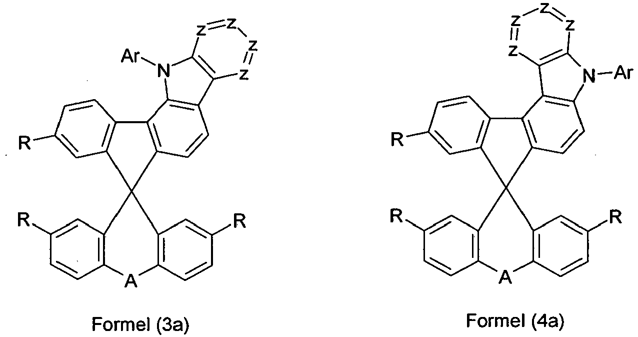 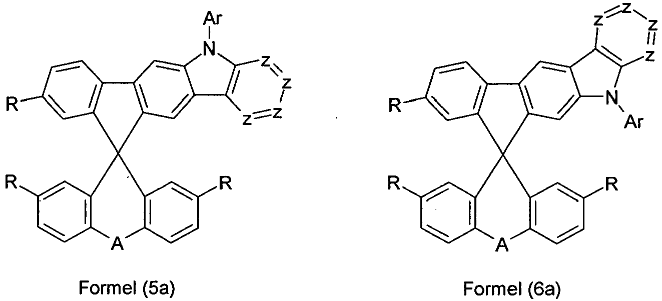 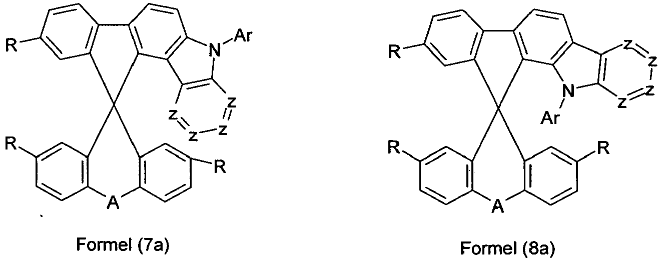 wobei die verwendeten Symbole die oben genannten Bedeutungen aufweisen.

In einer bevorzugten Ausführungsform der Erfindung steht Z für CR. Bevorzugte Ausführungsformen der vorliegenden Erfindung sind daher die Verbindungen der folgenden Formeln (3b) bis (8b), 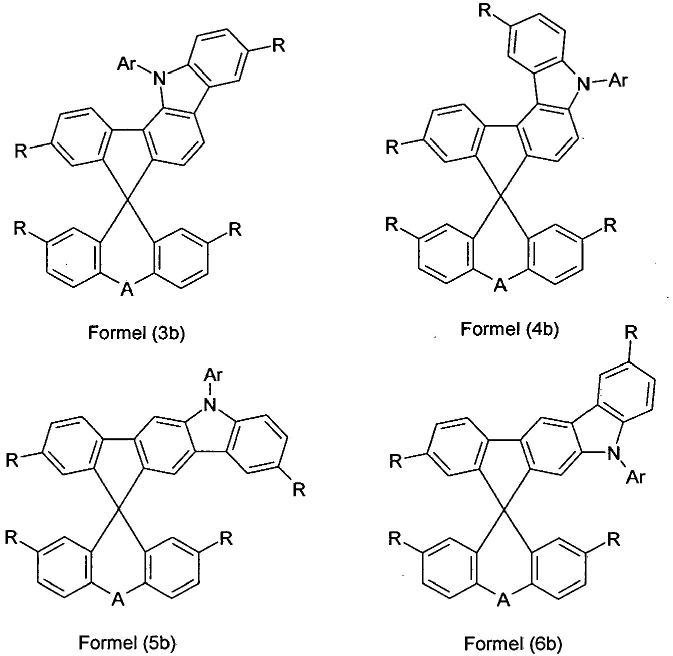 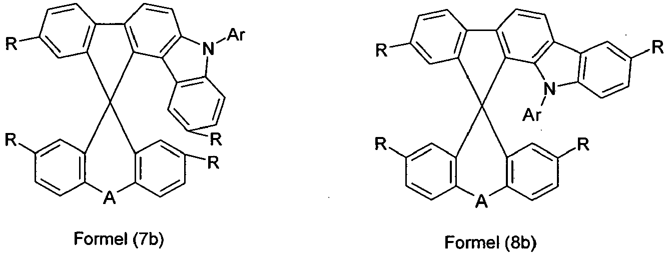 wobei die verwendeten Symbole die oben genannten Bedeutungen aufweisen.

In einer weiteren bevorzugten Ausführungsform der Erfindung steht in den Verbindungen der Formel (1), (3) bis (8), (3a) bis (8a) und (3b) bis (8b) das Symbol A für Sauerstoff.

Besonders bevorzugte Ausführungsformen der vorliegenden Erfindung sind die Verbindungen der folgenden Formeln (3c) bis (8c), 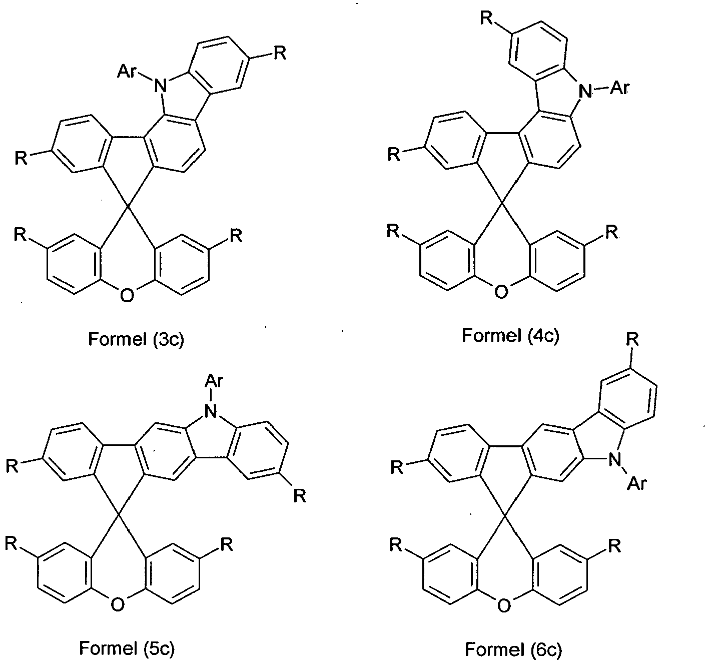 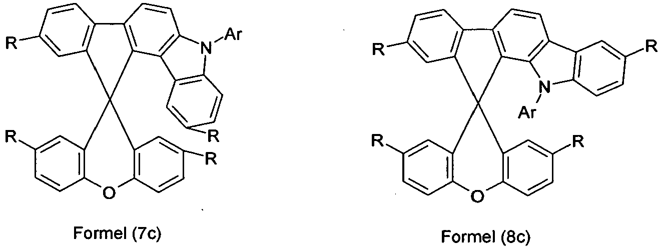 wobei die verwendeten Symbole die oben genannten Bedeutungen aufweisen.

Ganz besonders bevorzugte Ausführungsformen der vorliegenden Erfindung sind die Verbindungen der folgenden Formeln (3d) bis (8d), 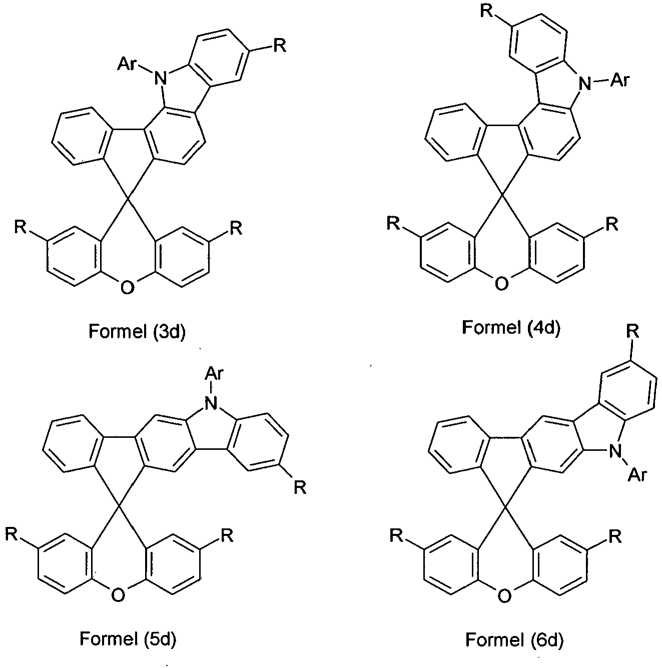 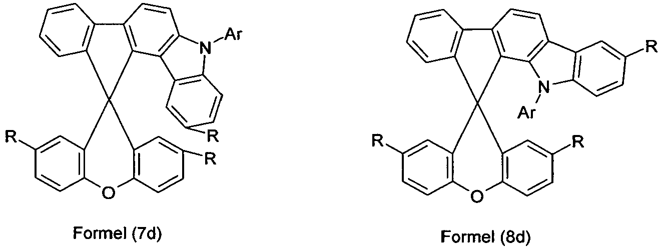 wobei die verwendeten Symbole die oben genannten Bedeutungen aufweisen.

In einer bevorzugten Ausführungsform der Erfindung ist Ar ist ein aromatisches oder heteroaromatisches Ringsystem mit 5 bis 24 aromatischen Ringatomen, bevorzugt mit 6 bis 12 aromatischen Ringatomen, welches jeweils durch einen oder mehrere Reste R substituiert sein kann. Geeignete Gruppen Ar sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 2-verknüpftem Fluoren, Spirobifluoren, insbesondere 2- oder 4-verknüpftem Spirobifluoren, Naphthalin, insbesondere 1- oder 2-verknüpftem Naphthalin, Indol, Benzofuran, Benzothiophen, Carbazol, insbesondere 2- oder 3-verknüpftem Carbazol, Dibenzofuran, insbesondere 1-, 2-, 3- oder 4-verknüpftem Dibenzofuran, Dibenzothiophen, insbesondere 1-, 2-, 3- oder 4-verknüpftem Dibenzothiophen, Indenocarbazol, Indolocarbazol, Pyridin, Pyrimidin, Pyrazin, Pyridazin, Triazin, Phenanthren, Triphenylen oder Kombinationen aus zwei oder drei dieser Gruppen, welche jeweils mit einem oder mehreren Resten R substituiert sein können.

Dabei sind die Gruppen Ar bevorzugt gewählt aus den Gruppen der folgenden Formeln Ar-1 bis Ar-56, 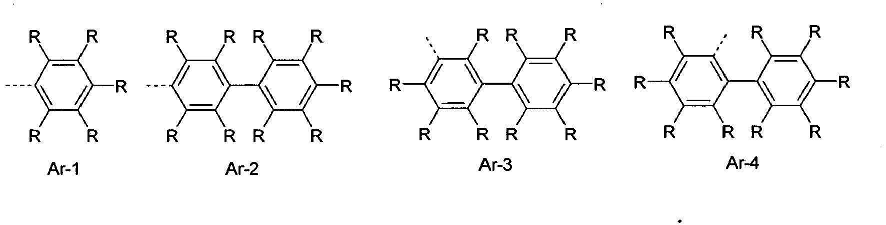 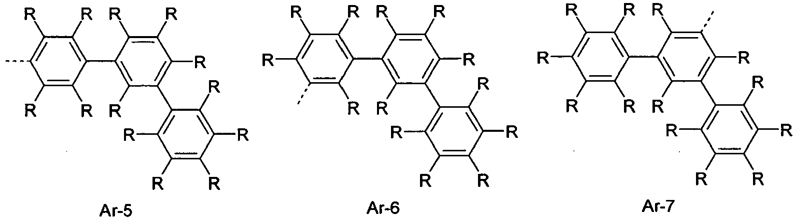 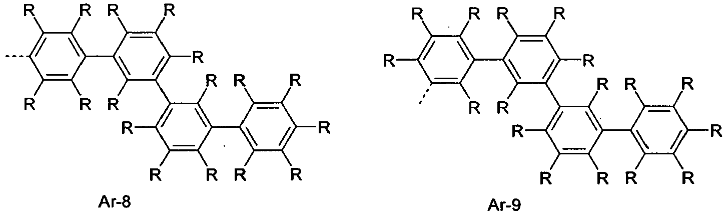 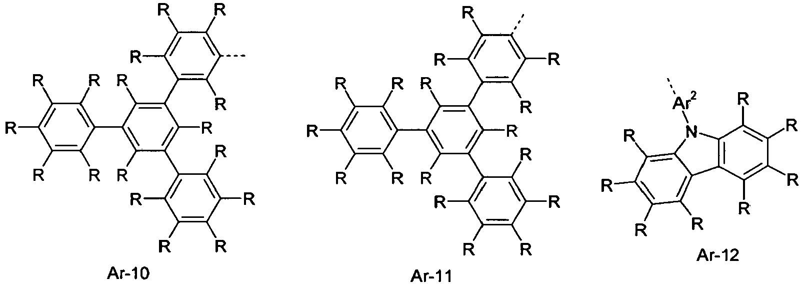 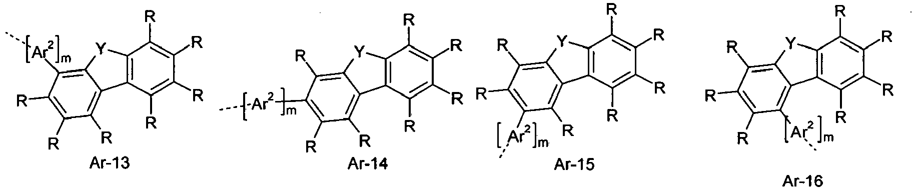 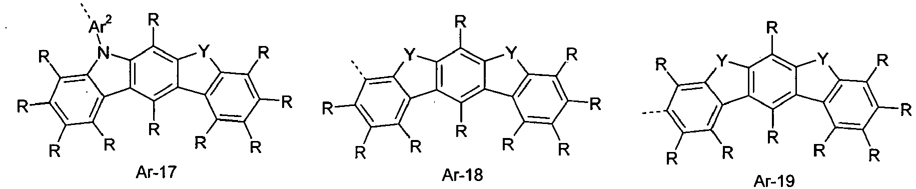 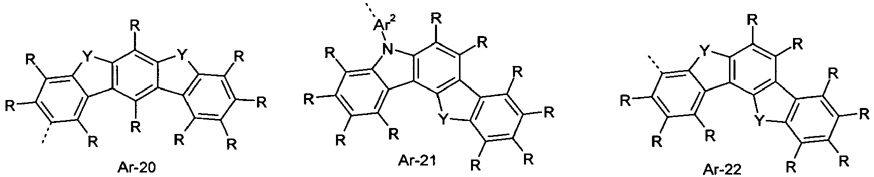 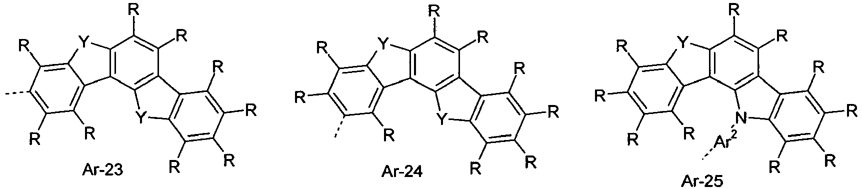 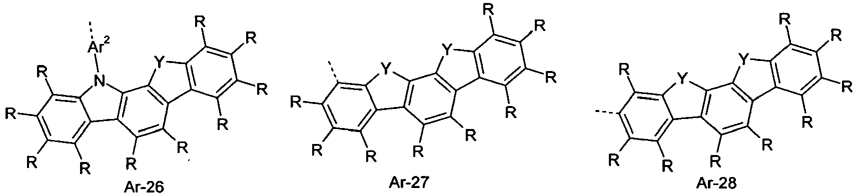 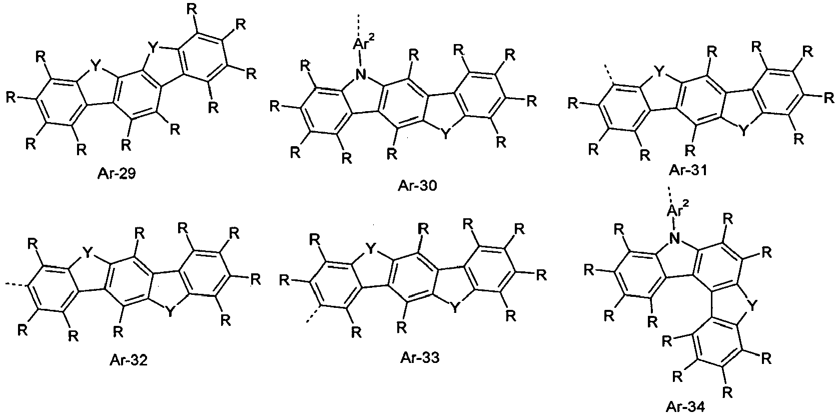 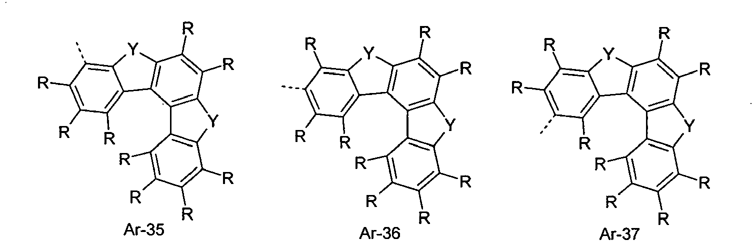 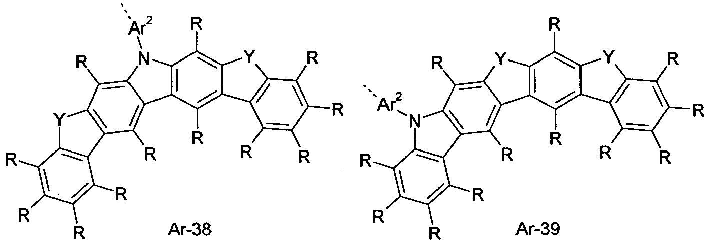 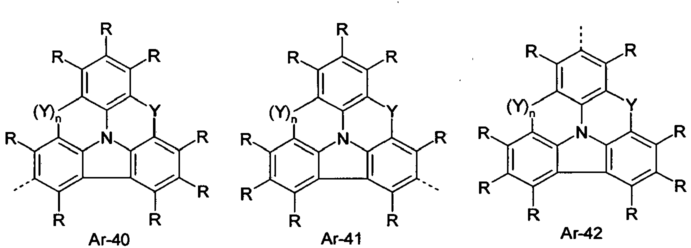 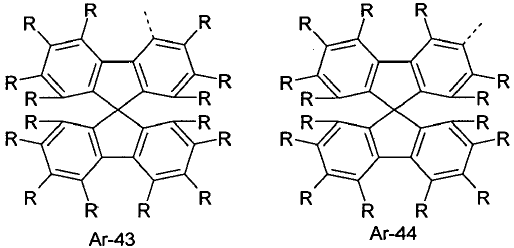 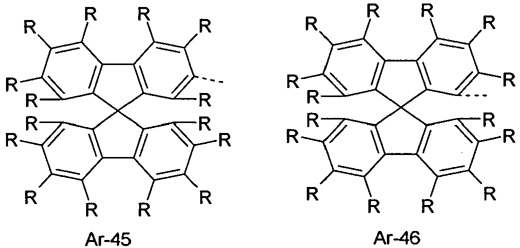 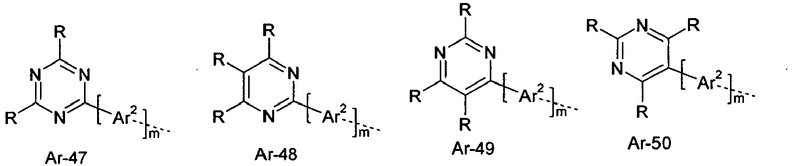 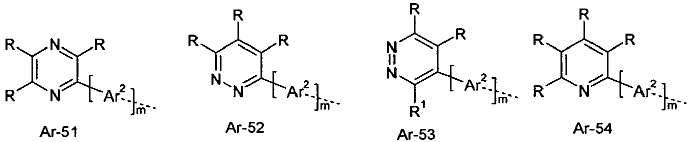 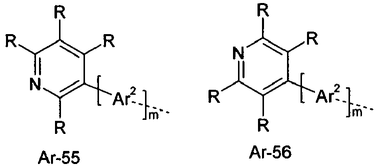 wobei R die oben genannten Bedeutungen aufweist, die gestrichelte Bindung die Bindung an den Stickstoff in Formel (2) bzw. in den bevorzugten Ausführungsformen darstellt und weiterhin gilt:
- Ar²: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R substituiert sein kann;
- Y: ist bei jedem Auftreten gleich oder verschieden CR₂, NR, O oder S;
- n: ist 0 oder 1, wobei n = 0 bedeutet, dass an dieser Position keine Gruppe Y gebunden ist und an den entsprechenden Kohlenstoffatomen statt dessen Reste R gebunden sind;
- m: ist 0 oder 1, wobei m = 0 bedeutet, dass die Gruppe Ar² nicht vorhanden ist und dass die entsprechende aromatische bzw. heteroaromatische Gruppe direkt an den Stickstoff in Formel (2) bzw. in den bevorzugten Ausführungsformen gebunden ist.

Wenn die oben genannten Gruppen für Ar mehrere Gruppen Y aufweisen, so kommen hierfür alle Kombinationen aus der Definition von Y in Frage. Bevorzugte Ausführungsformen sind dann solche, in denen eine Gruppe Y für NR und die andere Gruppe Y für CR₂ steht oder in denen beide Gruppen Y für NR stehen oder in denen beide Gruppen Y für O stehen. In einer besonders bevorzugten Ausführungsform der Erfindung steht in Gruppen Ar, die mehrere Gruppen Y aufweisen, mindestens eine Gruppe Y für CR₂ oder für NR.

Wenn Y für NR steht, steht der Substituent R, der an das Stickstoffatom gebunden ist, bevorzugt für ein aromatisches oder heteroaromatisches Ringsystem mit 5 bis 24 aromatischen Ringatomen, welches auch durch einen oder mehrere Reste R¹ substituiert sein kann. In einer besonders bevorzugten Ausführungsform steht dieser Substituent R gleich oder verschieden bei jedem Auftreten für ein aromatisches oder heteroaromatisches Ringsystem mit 6 bis 24 aromatischen Ringatomen, welches keine kondensierten Arylgruppen aufweist und welches keine kondensierten Heteroarylgruppen, in denen zwei oder mehr aromatische bzw. heteroaromatische 6-Ring-Gruppen direkt aneinander ankondensiert sind, aufweist, und welches jeweils auch durch einen oder mehrere Reste R¹ substituiert sein kann. Besonders bevorzugt sind Phenyl, Biphenyl, Terphenyl und Quaterphenyl mit Verknüpfungsmustern, wie vorne für Ar-1 bis Ar-11 aufgeführt, wobei diese Strukturen durch einen oder mehrere Reste R¹ substituiert sein können, bevorzugt aber unsubstituiert sind.

Wenn Y für CR₂ steht, stehen die Substituenten R, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 5 bis 24 aromatischen Ringatomen, welches auch durch einen oder mehrere Reste R¹ substituiert sein kann. Ganz besonders bevorzugt steht R für eine Methylgruppe oder für eine Phenylgruppe. Dabei können die Reste R auch miteinander ein Ringsystem bilden, was zu einem Spirosystem führt.

In einer weiteren bevorzugten Ausführungsform der Erfindung ist R am Grundgerüst der Verbindungen der Formel (1) sowie in den bevorzugten Ausführungsformen gleich oder verschieden bei jedem Auftreten ausgewählt aus der Gruppe bestehend aus H, D, F, CN, N(Ar¹)₂, C(=O)Ar¹, P(=O)(Ar¹)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, oder einem aromatischen oder heteroaromatischen Ringsystem mit 5 bis 30 aromatischen Ringatomen, das jeweils mit einem oder mehreren Resten R¹ substituiert sein kann. Besonders bevorzugt ist R gleich oder verschieden bei jedem Auftreten ausgewählt aus der Gruppe bestehend aus H, N(Ar¹)₂, einer geradkettigen Alkylgruppe mit 1 bis 6 C-Atomen, insbesondere mit 1 bis 4 C-Atomen, oder einer verzweigten oder cyclischen Alkylgruppe mit 3 bis 8 C-Atomen, insbesondere mit 3 bis 6 C-Atomen, die jeweils mit einem oder mehreren Resten R¹ substituiert sein kann, bevorzugt aber unsubstiutiert ist, oder einem aromatischen oder heteroaromatischen Ringsystem mit 5 bis 24 aromatischen Ringatomen, das jeweils mit einem oder mehreren Resten R¹ substituiert sein kann. Ganz besonders bevorzugt ist R gleich oder verschieden bei jedem Auftreten ausgewählt aus der Gruppe bestehend aus H, N(Ar¹)₂ oder einem aromatischen oder heteroaromatischen Ringsystem mit 5 bis 24 aromatischen Ringatomen, das jeweils mit einem oder mehreren Resten R¹ substituiert sein kann.

Wenn R für ein aromatisches oder heteroaromatisches Ringsystem steht, dann ist dieser Rest R bevorzugt gleich oder verschieden bei jedem Auftreten aus denselben Gruppen ausgewählt, wie oben als geeignete Gruppen für Ar angegeben, insbesondere aus den Gruppen Ar-1 bis Ar-56, an die statt den Substituenten R jedoch Substituenten R¹ gebunden sind.

Weitere geeignete Gruppen R sind Gruppen der Formel -[Ar⁵]_{q}-N(Ar³)(Ar⁴), wobei q für 0 oder 1 steht und Ar³, Ar⁴ und Ar⁵ gleich oder verschieden bei jedem Auftreten für ein aromatisches oder heteroaromatisches Ringsystem mit 5 bis 24 aromatischen Ringatomen steht, welches jeweils mit einem oder mehreren Resten R¹ substituiert sein kann.

Dabei können Ar⁵ und Ar³ für q = 1 miteinander und/oder Ar³ und Ar⁴ miteinander auch durch eine Einfachbindung oder durch eine Gruppe ausgewählt aus C(R¹)₂, NR¹, O oder S verbunden sein, bevorzugt durch eine Einfachbindung. Bevorzugt erfolgt die Verknüpfung von Ar⁶ und Ar³ miteinander bzw. von Ar³ und Ar⁴ miteinander jeweils ortho zur Position der Verknüpfung mit dem Stickstoffatom.

In einer Ausführungsform der Erfindung ist q = 1, und Ar⁵ und Ar³ sind miteinander durch eine Einfachbindung verbunden. In einer weiteren Ausführungsform der Erfindung ist q = 0 oder 1, und Ar³ und Ar⁴ sind miteinander durch eine Einfachbindung verbunden. In nochmals einer weiteren Ausführungsform der Erfindung ist q = 0 oder 1, und keine der Gruppen Ar³, Ar⁴ bzw. Ar⁶ sind miteinander verbunden.

Bevorzugt ist Ar⁵ ein aromatisches oder heteroaromatisches Ringsystem mit 6 bis 24 aromatischen Ringatomen, bevorzugt mit 6 bis 12 aromatischen Ringatomen, das jeweils mit einem oder mehreren Resten R¹ substituiert sein kann. Besonders bevorzugt ist Ar⁵ ausgewählt aus der Gruppe bestehend aus ortho-, meta- oder para-Phenylen oder ortho-, meta- oder para-Biphenyl, welche jeweils durch einen oder mehrere Reste R¹ substituiert sein können, bevorzugt aber unsubstituiert sind. Ganz besonders bevorzugt ist Ar⁵ eine unsubstituierte Phenylengruppe. Dies gilt insbesondere, wenn Ar⁵ mit Ar³ durch einen Einfachbindung verbunden ist.

Bevorzugt sind Ar³ und Ar⁴ gleich oder verschieden bei jedem Auftreten ein aromatisches oder heteroaromatisches Ringsystem mit 6 bis 24 aromatischen Ringatomen, das jeweils mit einem oder mehreren Resten R¹ substituiert sein kann. Besonders bevorzugte Gruppen Ar³ bzw. Ar⁴ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren, Triphenylen oder Kombinationen aus zwei, drei oder vier dieser Gruppen, welche jeweils mit einem oder mehreren Resten R¹ substituiert sein können. Besonders bevorzugt stehen Ar³ und Ar⁴ gleich oder verschieden bei jedem Auftreten für ein aromatisches Ringsystem mit 6 bis 24 aromatischen Ringatomen, welches durch einen oder mehrere Reste R¹ substituiert sein kann, insbesondere ausgewählt aus den Gruppen bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluören.

Besonders bevorzugte Gruppen der Formel -[Ar⁵]_{q}-N(Ar³)(Ar⁴) sind solche, in denen q = 1 ist und Ar⁵ für eine Phenylgruppe steht, die mit Ar³ durch eine Einfachbindung verbunden ist. Dadurch entsteht ein Carbazol bzw. ein Carbazolderivat.

In einer weiteren bevorzugten Ausführungsform der Erfindung handelt es sich bei den Verbindungen der Formel (1) bzw. den bevorzugten Ausführungsformen um bipolare Verbindungen, also Verbindungen, die sowohl elektronentransportierende wie auch lochtransportierende Einheiten enthalten. Dabei ist es bevorzugt, wenn die Gruppe Ar eine elektronentransportierende Einheit darstellt, beispielsweise eine Einheit gemäß einer der oben genannten Formeln Ar-47 bis Ar-56, und wenn mindestens ein Rest R, insbesondere ein Rest R an der Einheit der Formel (2) eine lochtransportierende Einheit darstellt, insbesondere eine Einheit der oben genannten Formel -[Ar⁵]_{q}-N(Ar³)(Ar⁴).

Dabei haben in Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Wenn die Verbindungen der Formel (1) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es weiterhin bevorzugt, wenn die Verbindung keine kondensierten Aryl- bzw. Heteroarylgruppen enthält, in denen mehr als zwei Sechsringe direkt aneinander ankondensiert sind. Insbesondere ist es bevorzugt, dass die Reste R, R¹ und Ar¹ bis Ar⁵ keine kondensierten Aryl- bzw. Heteroarylgruppen enthalten, in denen zwei oder mehr Sechsringe direkt aneinander ankondensiert sind.

Die oben genannten bevorzugten Ausführungsformen können beliebig miteinander kombiniert werden. In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die in der folgenden Tabelle aufgeführten Verbindungen.

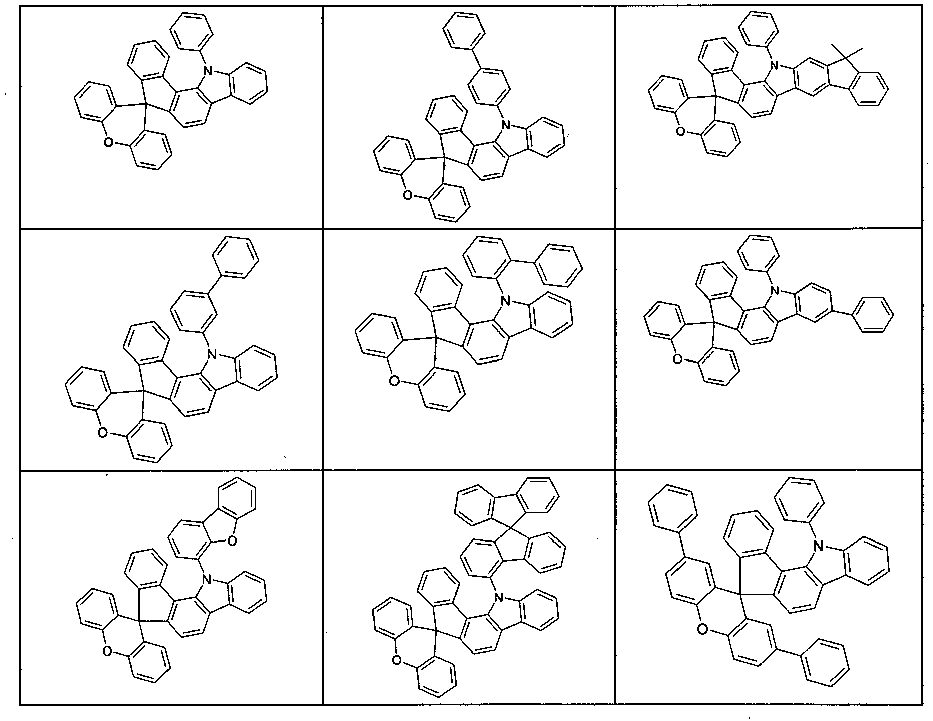

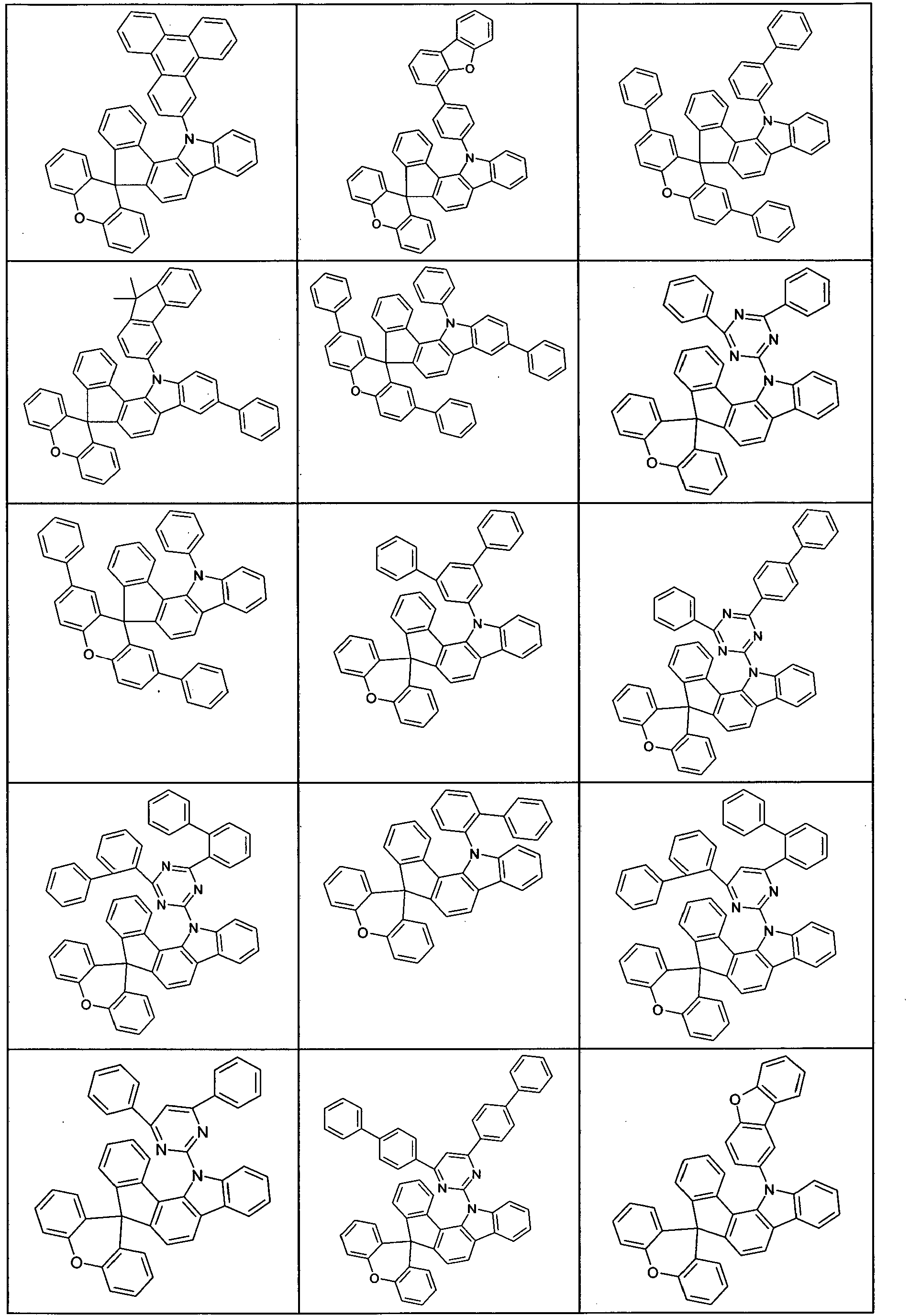

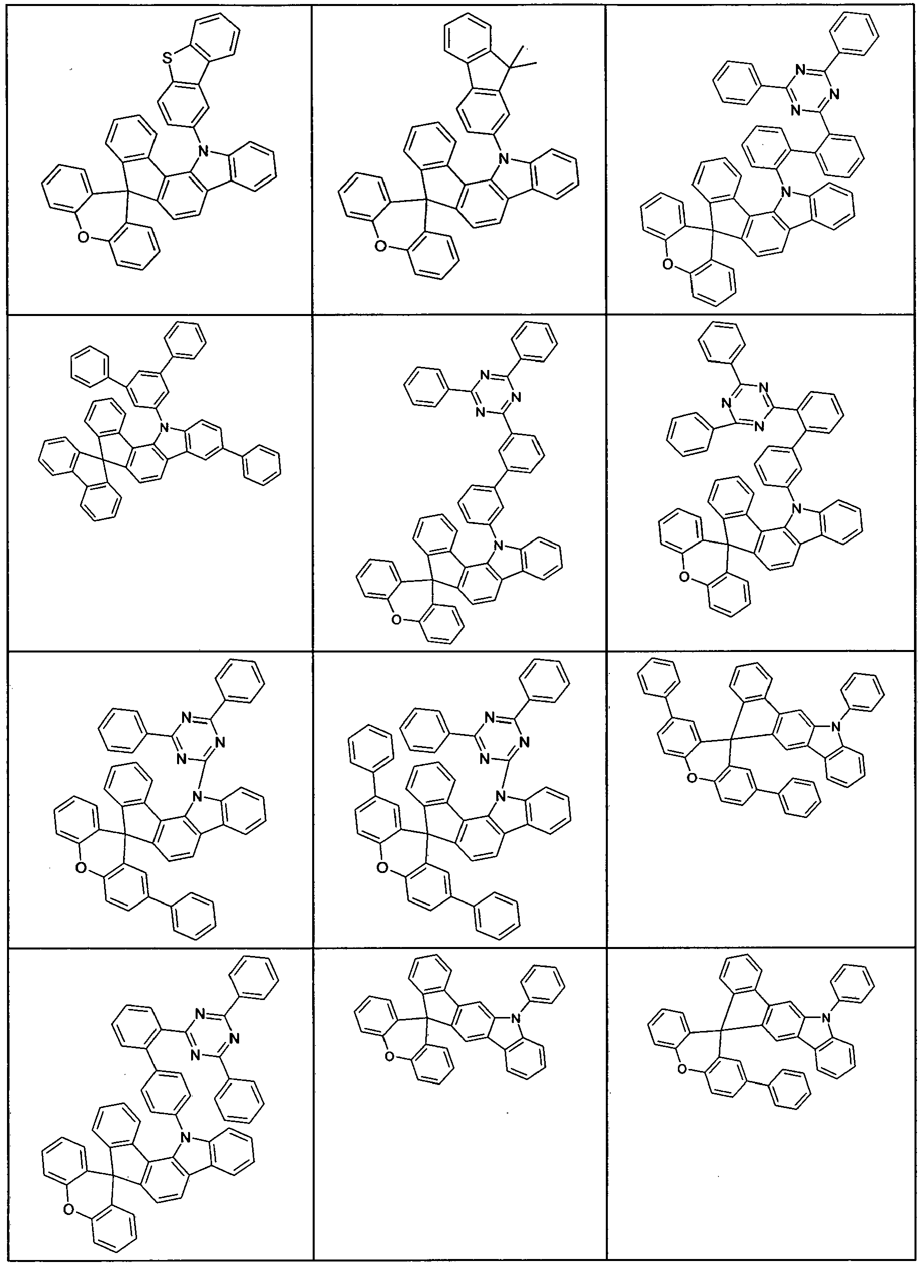

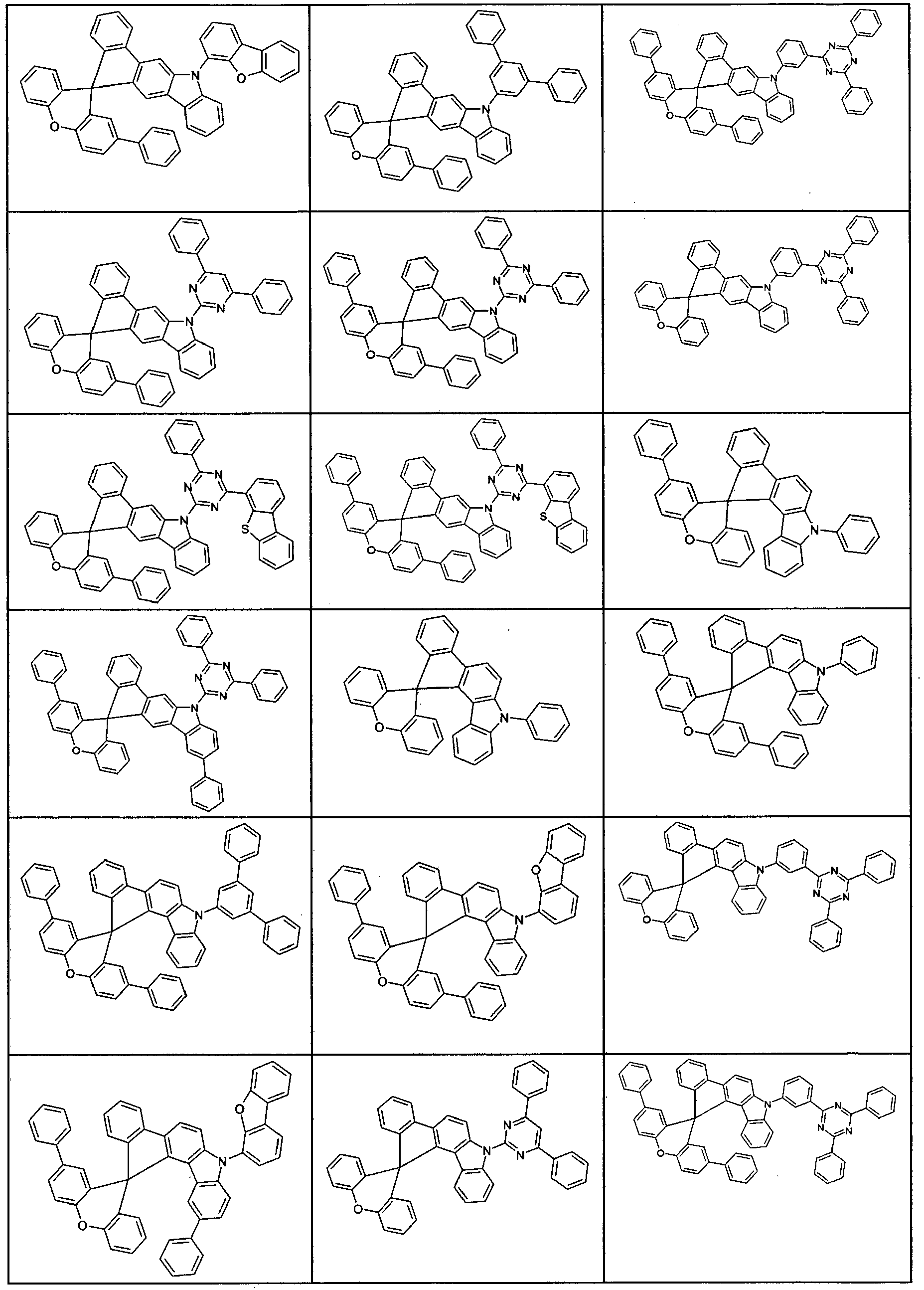

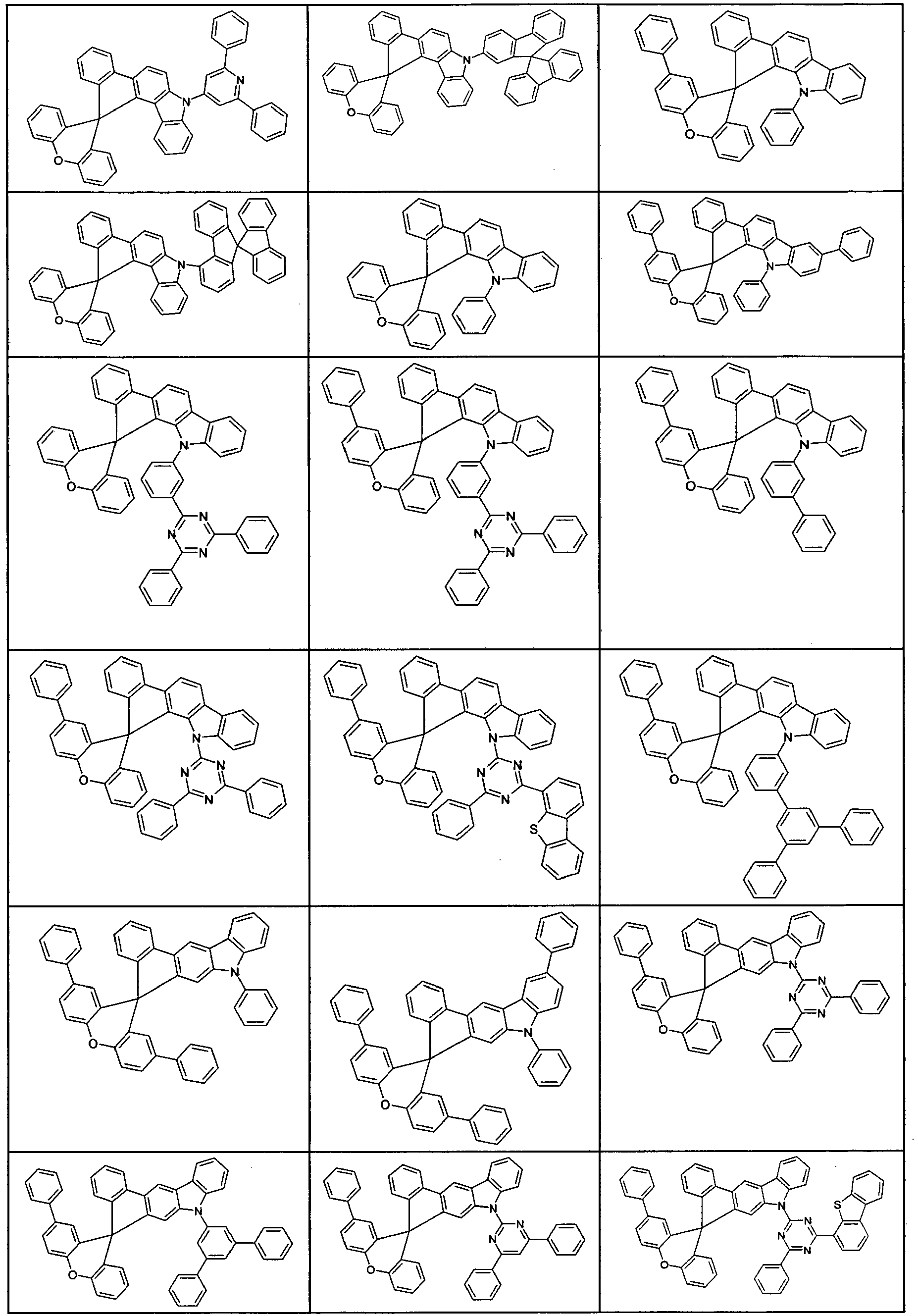

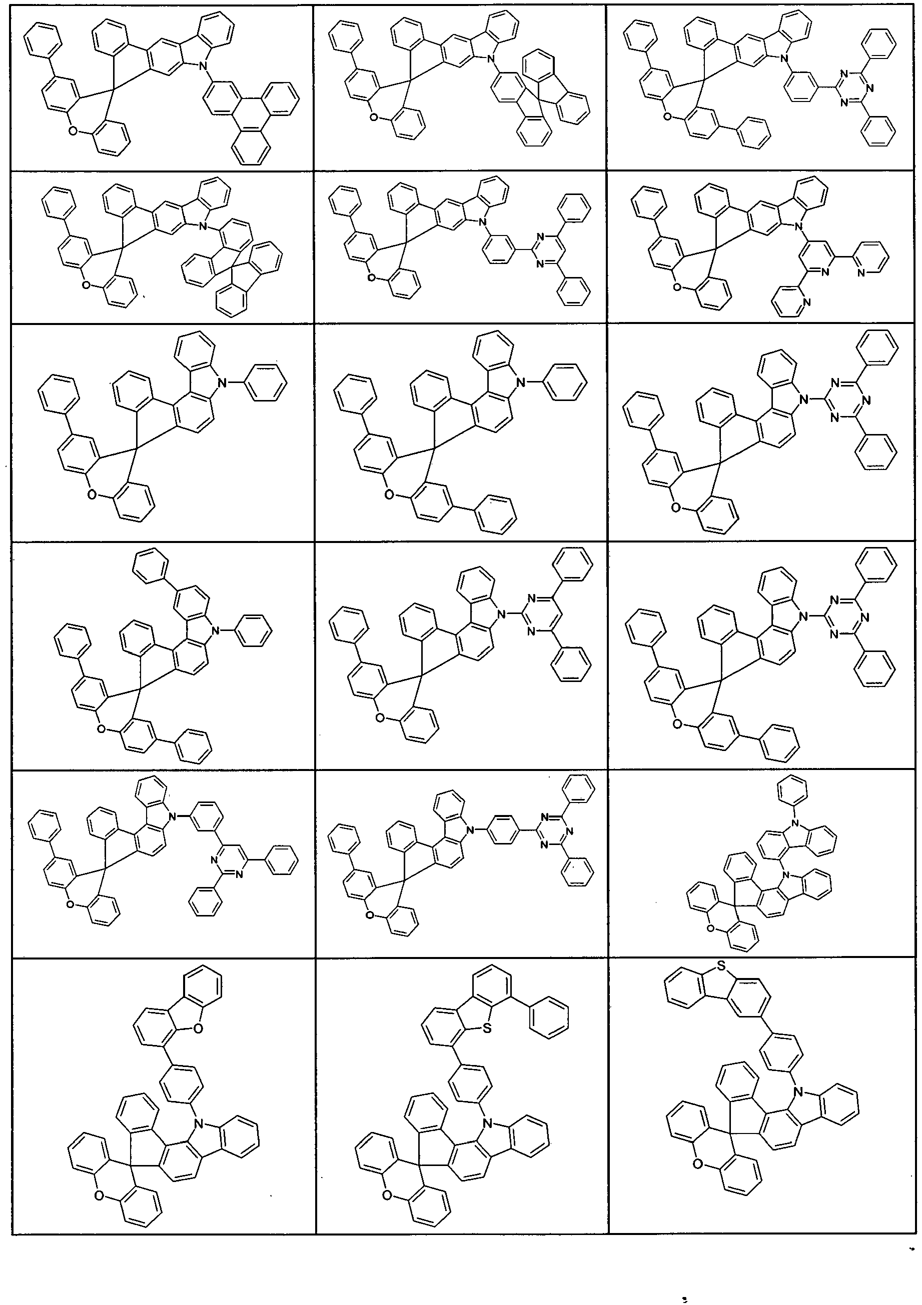

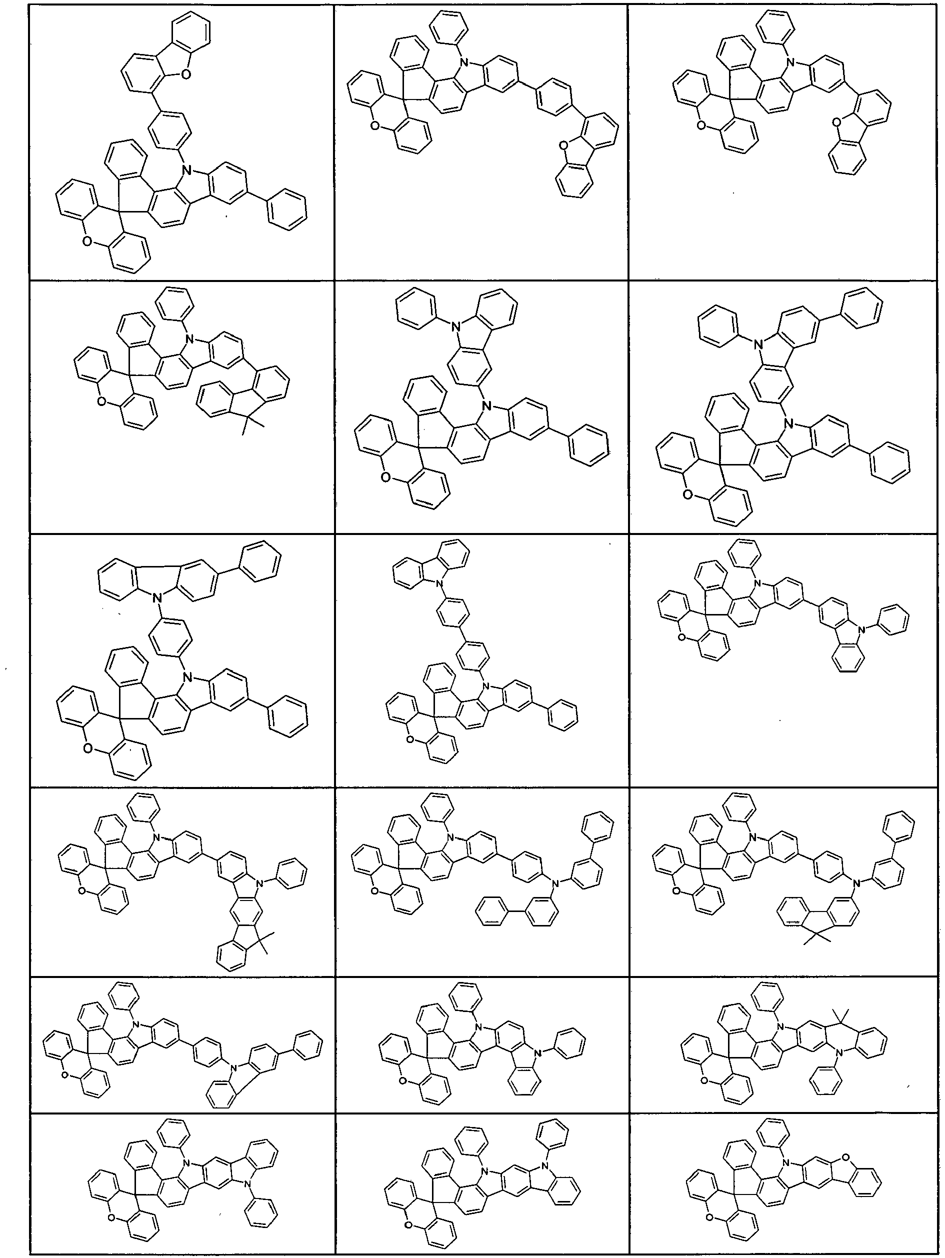

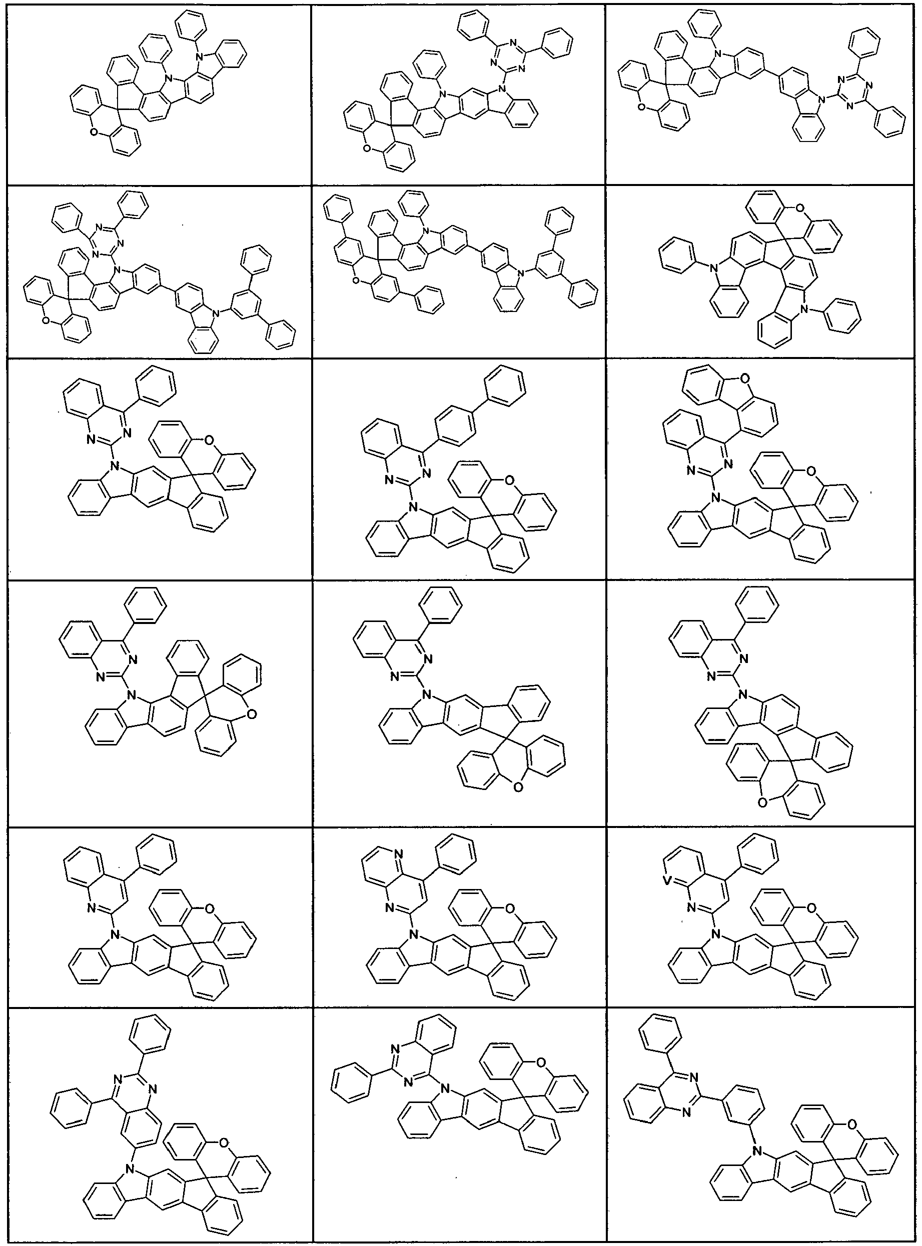

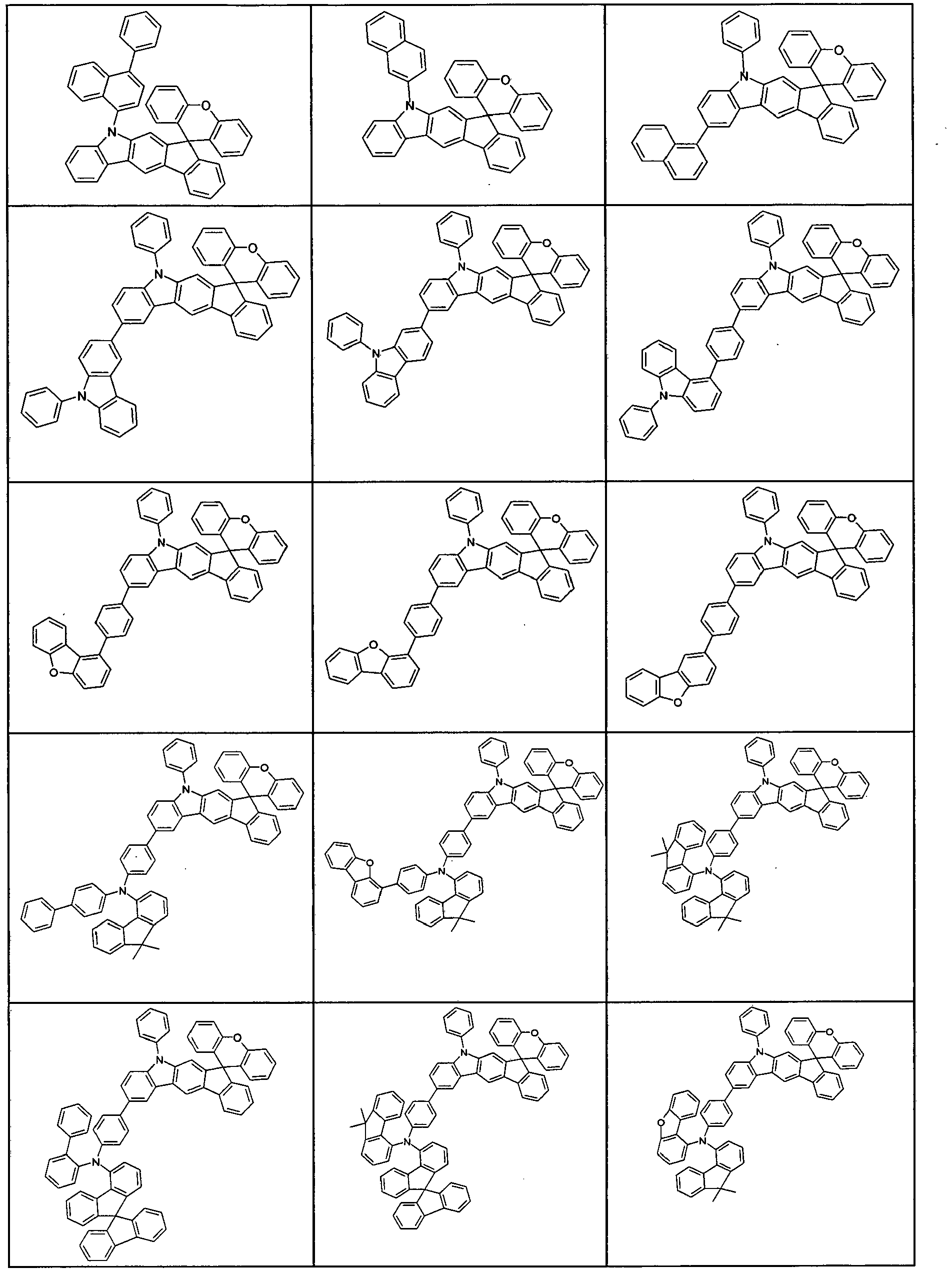

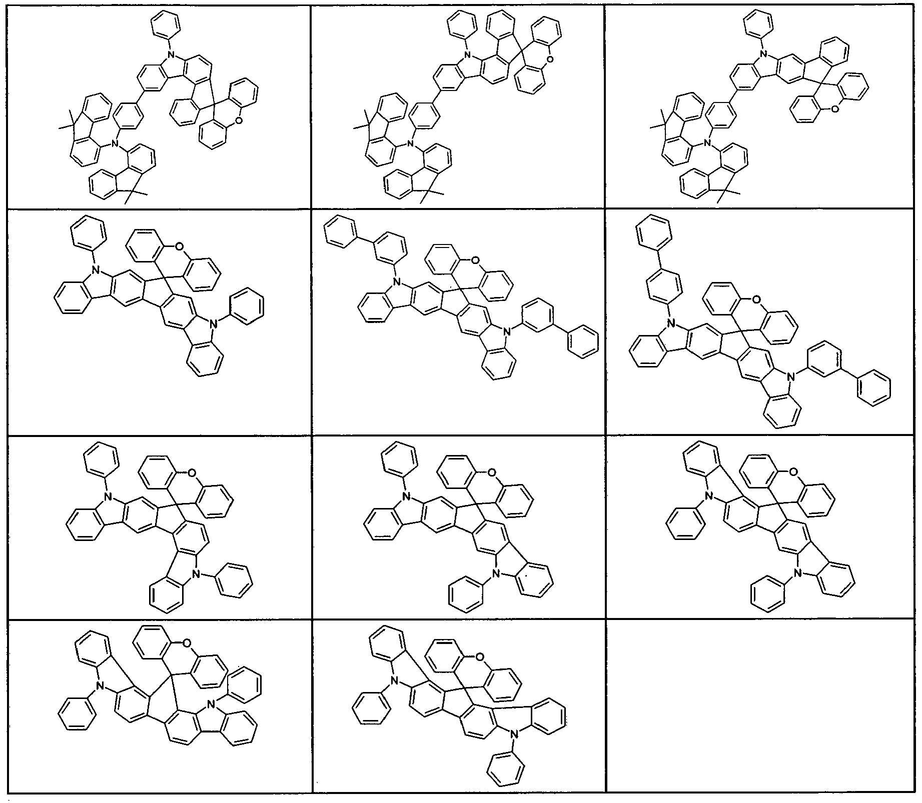

Die Grundstruktur der erfindungsgemäßen Verbindungen kann nach dem in Schema 1 skizzierten Weg dargestellt werden. Die Einführung der Gruppe Ar kann gemäß Schema 2 erfolgen.

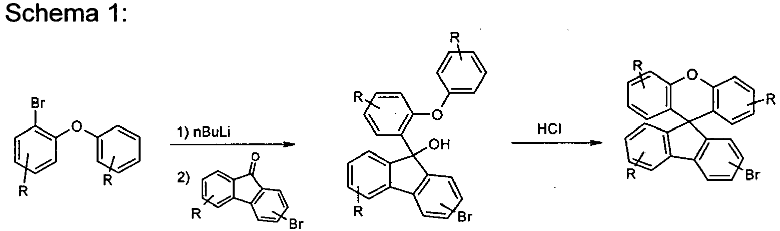

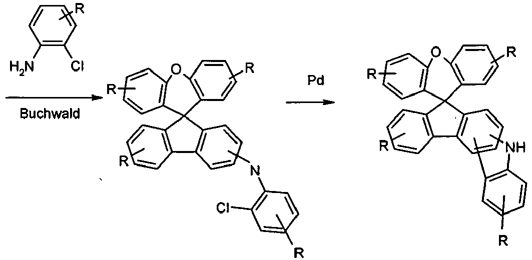

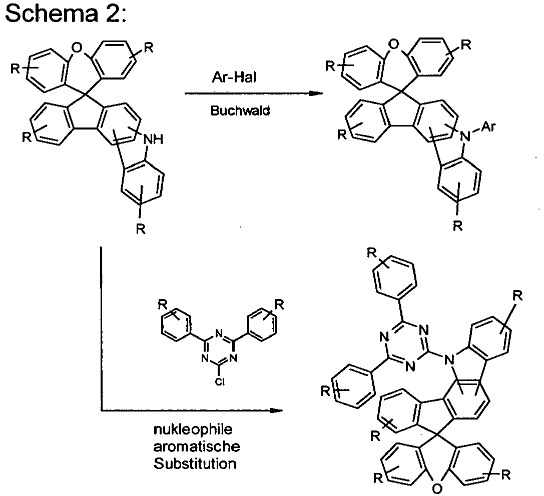

Dabei kann die Synthese des Grundgerüsts von einem halogensubstituierten Diphenylether oder analog mit einem halogensubstitutierten Diphenylthioether erfolgen, welches lithiiert und mit einem halogensubstituierten Fluorenon umgesetzt wird, gefolgt von der Ringschlussreaktion zur entsprechenden Spiroverbindung unter Säureeinfluss. Dieses wird mit einem ortho-Halogenaminobenzol in einer C-N-Kupplungsreaktion, beispielsweise unter Pd- oder Cu-Katalyse, umgesetzt. In den oben genannten Reaktionen ist das Halogen bevorzugt Cl, Br oder I, insbesondere Br. Der Ringschluss zum entsprechenden Carbazolderivat erfolgt durch eine intramolekulare Pd-katalysierte Kupplungsreaktion.

Verbindungen der Formel (1) erhält man durch eine nukleophile aromatische Substitutionsreaktion oder durch eine Pd-katalysierte Kupplungsreaktion, beispielsweise eine Hartwig-Buchwald-Kupplung oder Ullmann-Kupplung, mit einer Gruppe Ar, die mit einer entsprechenden Abgangsgruppe, insbesondere Cl oder Br, substituiert ist.

Ein weiterer Gegenstand der vorliegenden Erfindung ist ein Verfahren zur Herstellung einer Verbindung gemäß Formel (1), umfassend die Reaktionsschritte:
a) Synthese der Grundgerüsts der Verbindung (1), die noch keine Gruppe Ar enthält; und
b) Umsetzung der Grundgerüsts aus a) in einer C-N-Kupplung, wie Buchwald- oder Ullmann-Kupplung, oder in einer nukleophilen aromatischen Substitutionsreaktion zur Einführung der Gruppe Ar.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-lsopropylnaphthalin, Pentylbenzol, Hexylbenzol, Heptylbenzol, Octylbenzol, 1,1-Bis(3,4-dimethylphenyl)ethan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h, in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich bei der erfindungsgemäßen organischen Elektrolumineszenzvorrichtung auch um eine Tandem-OLED handeln, insbesondere auch für weiß emittierende OLEDs.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen als Matrixmaterial für phosphoreszierende Emitter in einer emittierenden Schicht. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält. Weiterhin kann die erfindungsgemäße Verbindung auch in einer Elektronentransportschicht und/oder in einer Lochtransportschicht und/oder in einer Exzitonenblockierschicht und/oder in einer Lochblockierschicht eingesetzt wird

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, oder Triphenylenderivate, z. B. gemäß WO 2012/048781. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben.

Insbesondere eignen sich in Kombination mit der erfindungsgemäßen Verbindung als Co-Matrix-Material Verbindungen, welche eine große Bandlücke aufweisen und selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in der WO 2009/124627 oder in der WO 2010/006680.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 2005/033244, WO 2005/019373, US 2005/0258742, WO 2010/086089, WO 2011/044988, WO 2011/157339, WO 2012/007086, WO 2012/163471, WO 2013/000531 und WO 2013/020631, WO 2014/008982, WO 2014/023377 entnommen werden. Weiterhin eignen sich beispielsweise die in den nicht offen gelegten Anmeldungen EP 12008582.4, EP 13003484.6, EP 13003485.3, EP 13004552.9, EP 14000345.0 und EP 14000417.7 offenbarten Metallkomplexe. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Die erfindungsgemäßen Verbindungen sind insbesondere auch geeignet als Matrixmaterialien für phosphoreszierende Emitter in organischen Elektrolumineszenzvorrichtungen, wie sie z. B. in WO 98/24271, US 2011/0248247 und US 2012/0223633 beschrieben sind. In diesen mehrfarbigen Display-Bauteilen wird eine zusätzliche blaue Emissionsschicht vollflächig auf alle Pixel, auch diejenigen mit einer von Blau verschiedenen Farbe, aufgedampft. Dabei wurde überraschend gefunden, dass die erfindungsgemäßen Verbindungen, wenn sie als Matrixmaterialien für das rote und/oder grüne Pixel eingesetzt werden, zusammen mit der aufgedampften blauen Emissionsschicht zu weiterhin sehr guter Emission führen.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen Verbindungen, eingesetzt als Matrixmaterial für phosphoreszierende Emitter, führen zu langen Lebensdauern.
2. Die erfindungsgemäßen Verbindungen führen zu hohen Effizienzen. Dies gilt insbesondere, wenn die Verbindungen als Matrixmaterial für einen phosphoreszierenden Emitter eingesetzt werden.
3. Die erfindungsgemäßen Verbindungen führen zu geringen Betriebsspannungen. Dies gilt insbesondere, wenn die Verbindungen als Matrixmaterial für einen phosphoreszierenden Emitter eingesetzt werden.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegeben Nummern sind die entsprechenden CAS-Nummern.

### a) Spiro-[2-brom-9H-fluoren-9,9'-(9H)-xanthen]

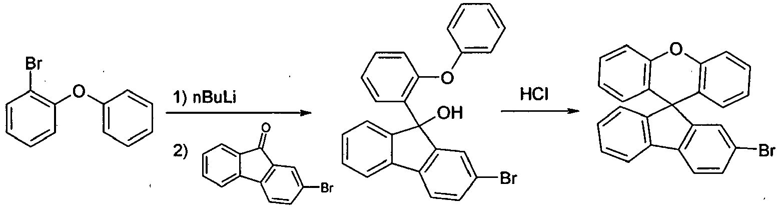

31.7 g (127 mmol) 1-Brom-2-diphenylether werden in einem ausgeheizten Kolben in 400 mL getrocknetem THF gelöst. Die Reaktionsmischung wird auf -78 °C gekühlt. Bei dieser Temperatur werden 55 mL einer 2.5 M-Lösung n-Butyllithium in Hexan (127 mmol) langsam zugetropft. Der Ansatz wird 1 h bei -70 °C nachgerührt. Anschließend werden 30 g 2-Bromfluorenon (116 mmol) in 100 ml THF gelöst und bei -70 °C zugetropft. Nach beendeter Zugabe wird die Reaktionsmischung langsam auf Raumtemperatur erwärmen lassen, mit NH₄Cl gequescht und anschließend am Rotationsverdampfer eingeengt. Die einrotierte Lösung wird vorsichtig mit 300 ml Essigsäure versetzt. Anschließend werden 50 ml rauchende HCl zugegeben. Der Ansatz wird 6 h auf 75 °C erhitzt. Dabei fällt ein weißer Feststoff aus. Der Ansatz wird nun auf Raumtemperatur abkühlen lassen, der ausgefallene Feststoff wird abgesaugt und mit Methanol gewaschen. Ausbeute: 45 g (95%)

Analog dazu werden folgende Verbindungen hergestellt:

| | **Edukt 1** | **Edukt 2** | **Produkt** | **Ausbeute** |
|---|---|---|---|---|
| **a1** | 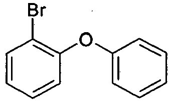 | 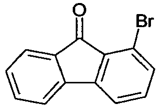 | 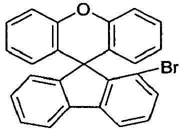 | 77% |
| **a2** | 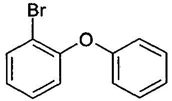 | 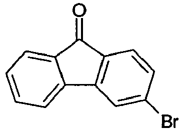 | 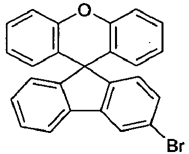 | 65% |
| **a3** | 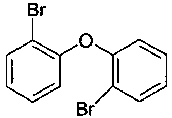 | 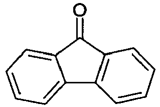 | 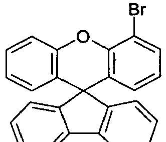 | 73% |

### b) 2-Chlorphenyl-4-spiro-[9H-fluoren-9,9'-(9H)-xanthenyl-amin

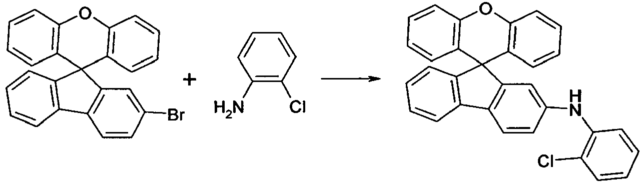

62.6 g (137 mmol) Spiro-[2-brom-9*H*-fluoren-9,9'-(9*H*)-xanthen], 17.9 g (140 mmol) 2-Chloranilin, 68.2 g (710 mmol) Natrium-tert-butylat, 613 mg (3 mmol) Palladium(II)acetat und 3.03 g (5 mmol) dppf werden in 1.3 L Toluol gelöst und 5 h unter Rückfluss gerührt. Das Reaktionsgemisch wird auf Raumtemperatur abgekühlt, mit Toluol erweitert und über Celite filtriert. Das Filtrat wird im Vakuum eingeengt und der Rückstand aus Toluol / Heptan kristallisiert. Das Produkt wird als farbloser Feststoff isoliert. Ausbeute: 58.4 g (127 mmol) 84% d.Th.

Analog können folgende Verbindungen hergestellt werden:

| | **Edukt 1** | **Edukt 2** | **Produkt** | **Ausbeute** |
|---|---|---|---|---|
| b1 | 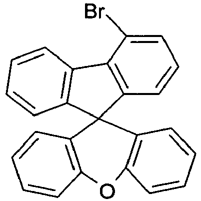 | 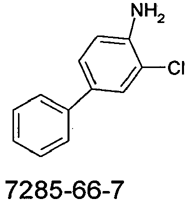 | 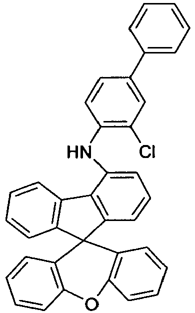 | 80% |
| b2 | 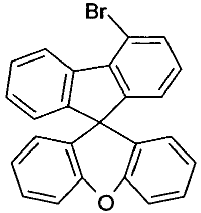 | 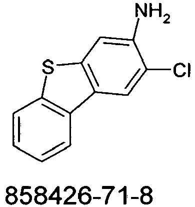 | 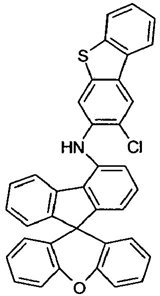 | 73% |
| b3 | 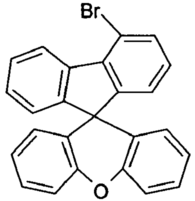 | 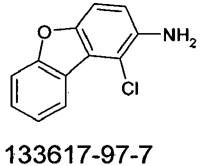 | 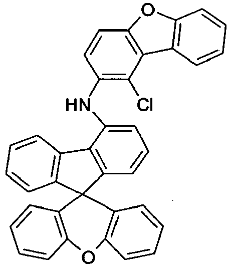 | 64% |
| b4 | 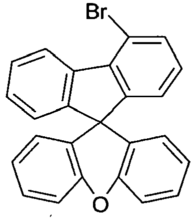 | 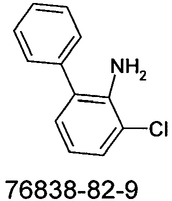 | 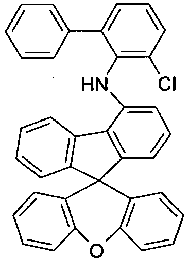 | 56% |
| b5 | 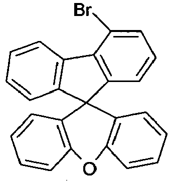 | 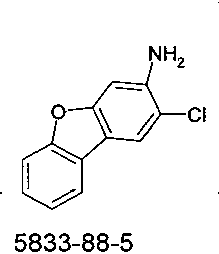 | 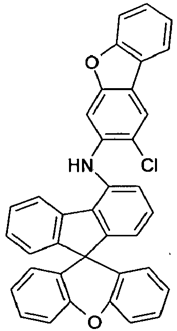 | 69% |
| b6 | 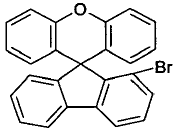 | 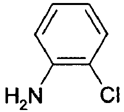 | 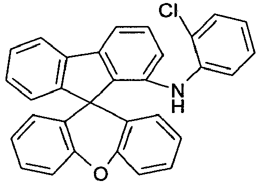 | 64% |
| b7 | 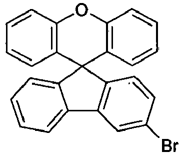 | 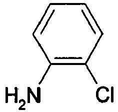 | 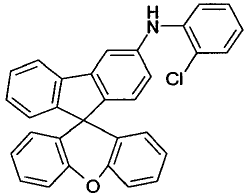 | 60% |
| b8 | 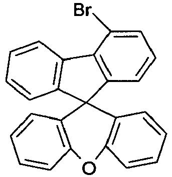 | 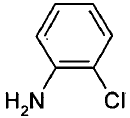 | 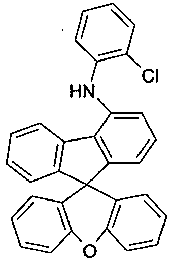 | 64% |

### c) Cyclisierung

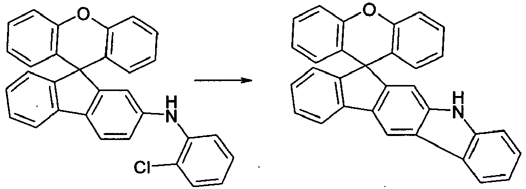

46.6 g (102 mmol) (2-Chlorphenyl)-4-spiro-9,9'-bifluorenyl-amin, 56 g (409 mmol) Kaliumcarbonat, 4.5 g (12 mmol) Tricyclohexylphosphintetrafluoroborat und 1.38 g (6 mmol) Palladium(II)acetat werden in 500 mL Dimethylacetamid suspendiert und 6 h unter Rückfluss gerührt. Nach Erkalten erweitert man die Reaktionmischung mit 300 ml Wasser, rührt 30 min. nach, trennt die organische Phase ab, filtriert diese über ein kurzes Celite-Bett und entfernt dann das Lösungsmittel im Vakuum. Das Rohprodukt wird mit Toluol heiß extrahiert und aus Toluol umkristallisiert. Das Produkt wird als beigefarbener Feststoff isoliert. Ausbeute: 33 g (78 mmol), entsprechend 77 % d.Th.

Analog können folgende Verbindungen hergestellt werden:

| | **Edukt** | **Produkt** | **Ausbeute** |
|---|---|---|---|
| c1 | 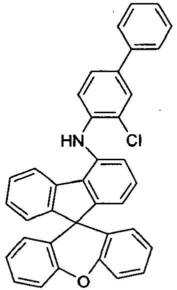 | 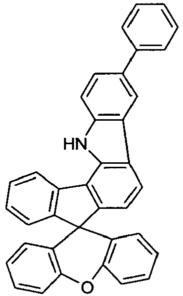 | 71% |
| c2 | 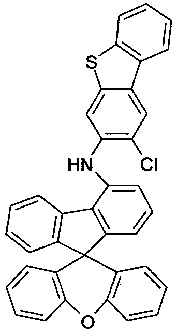 | 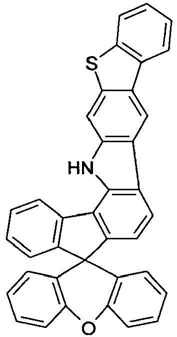 | 78% |
| c3 | 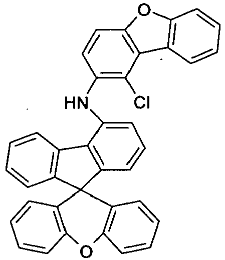 | 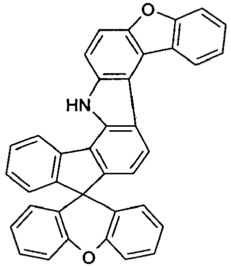 | 73% |
| c4 | 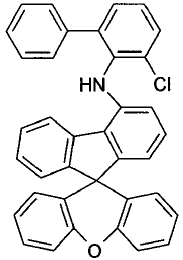 | 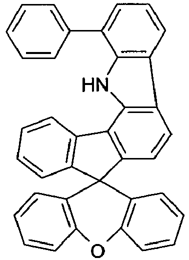 | 70% |
| c5 | 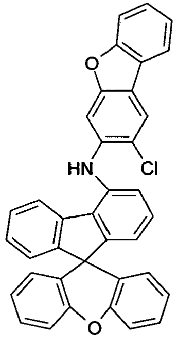 | 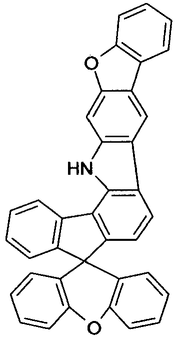 | 69% |
| c6 | 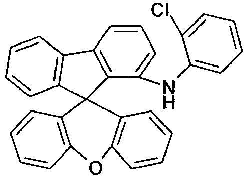 | 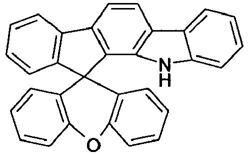 | 58% |
| c7 | 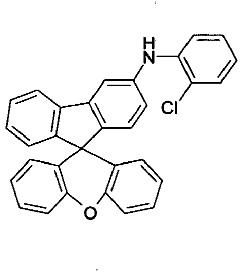 | 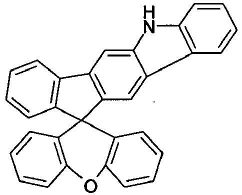 | 60% |
| | | 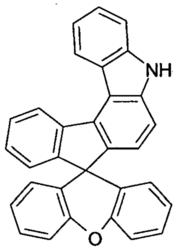 | 30% |
| c8 | 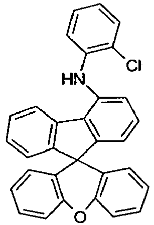 | 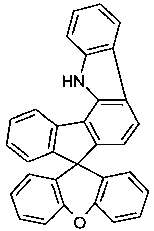 | 76% |

### d) Nukleophile aromatische Substitution

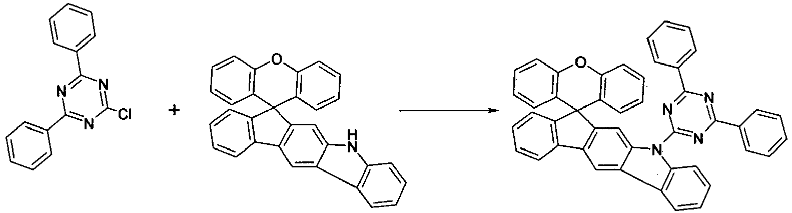

4.2 g NaH 60%ig in Mineralöl (106 mmol) werden in 300 mL Dimethylformamid unter Schutzatmosphäre gelöst. 46 g (106 mmol) Indeno[1,2-*a*]-carbazolderivat (aus c) werden in 250 mL DMF gelöst und zu der Reaktionsmischung zugetropft. Nach 1 h bei Raumtemperatur wird eine Lösung von 2-Chlor-4,6-diphenyl-[1,3,5]-triazin (34.5 g, 122 mmol) in 200 mL THF zugetropft. Das Reaktionsgemisch wird 12 h bei Raumtemperatur gerührt. Nach dieser Zeit wird das Reaktionsgemisch auf Eis gegossen. Der dabei ausgefallende Feststoff wird nach Erwärmen auf Raumtemperatur filtriert und mit Ethanol und Heptan gewaschen. Der Rückstand wird mit Toluol heiß extrahiert, aus Toluol/n-Heptan umkristallisiert und abschließend im Hochvakuum sublimiert.Die Reinheit beträgt 99.9%. Ausbeute an Produkt d: 30 g (46 mmol), entsprechend 43 % d.Th.

Analog können folgende Verbindungen hergestellt werden:

| | **Edukt1** | **Edukt 2** | **Produkt** | **Ausbeute** |
|---|---|---|---|---|
| 1d | 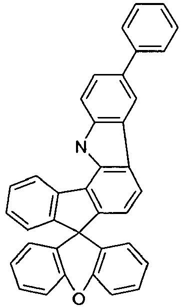 | 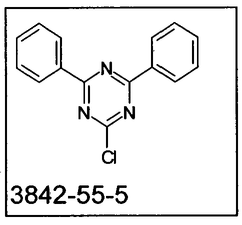 | 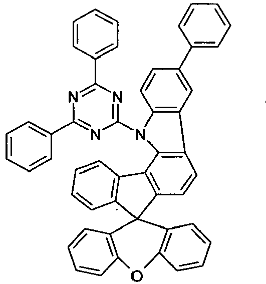 | 40% |
| 2d | 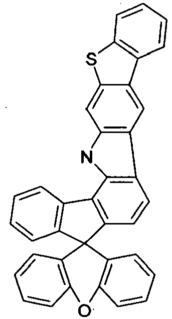 | 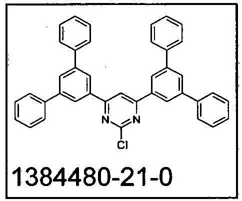 | 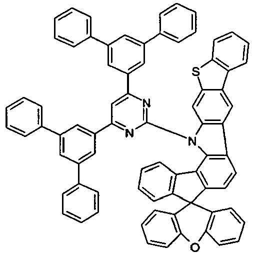 | 39% |
| 3d | 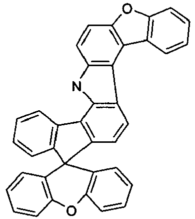 | 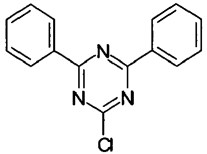 | 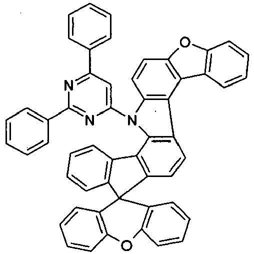 | 44% |
| 4d | 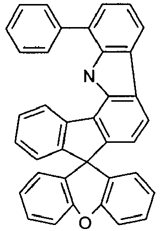 | 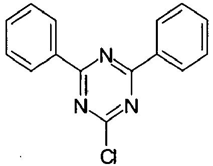 | 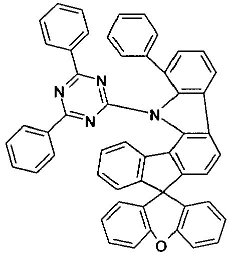 | 31% |
| 5d | 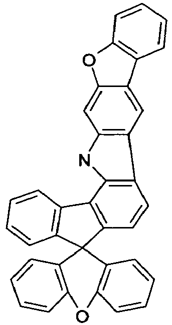 | 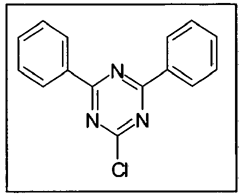 | 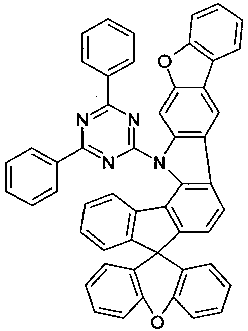 | 43% |
| 6d | 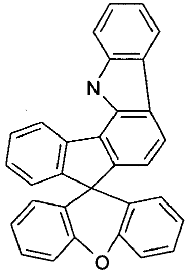 | 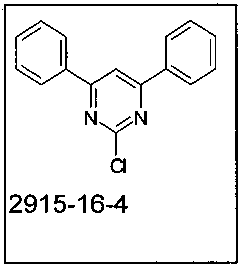 | 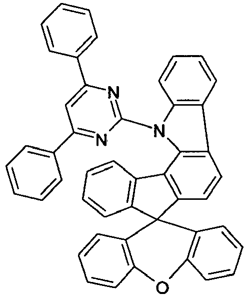 | 41% |
| 7d | 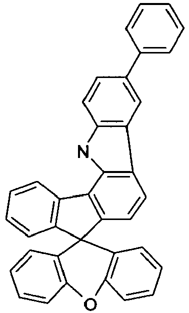 | 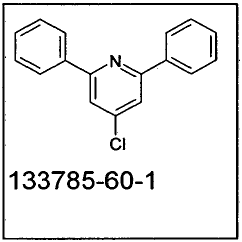 | 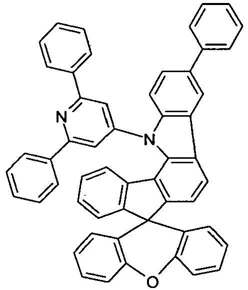 | 39% |
| 8d | 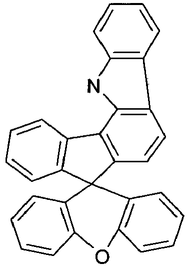 | 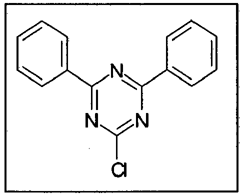 | 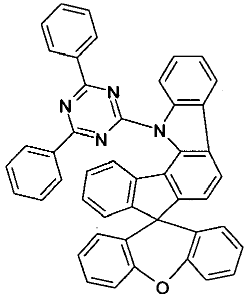 | 46% |
| 9d | 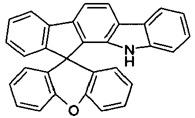 | 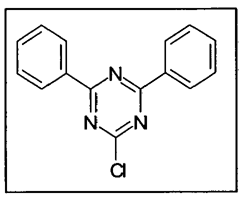 | 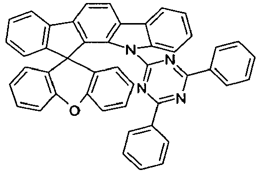 | 40% |
| 10d | 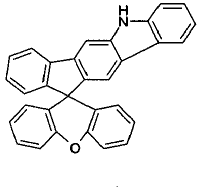 | 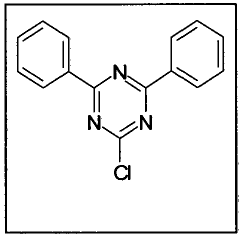 | 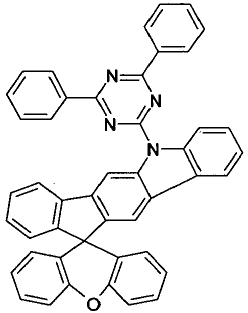 | 51% |

### e) Buchwald-Reaktion

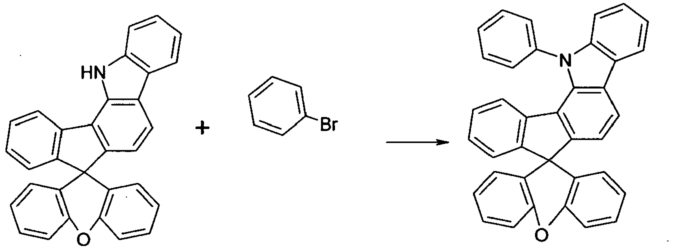

44.6 g (106 mmol) Indeno[1,2-*a*]carbazol-Derivat (aus c), 17.9 g (114 mmol) Brombenzol und 30.5 g NaOtBu werden in 1.5 L p-Xylol suspendiert. Zu dieser Suspension werden 0.5 g (2.11 mmol) Pd(OAc)₂ und 1.6 ml einer 1M Tri-tert-butylphosphin-Lösung gegeben. Die Reaktionsmischung wird 16 h unter Rückfluss erhitzt. Nach Erkalten wird die organische Phase abgetrennt, dreimal mit 200 mL Wasser gewaschen und anschließend zur Trockene eingeengt. Der Rückstand wird mit Toluol heiß extrahiert, aus Toluol umkristallisiert und abschließend im Hochvakuum sublimiert. Die Reinheit beträgt 99.9% bei einer Ausbeute an Produkt e von 22.6 g (45 mmol; 43%).

Analog können folgende Verbindungen hergestellt werden:

| | | | | |
|---|---|---|---|---|
| | **Edukt1** | **Edukt 2** | **Produkt** | **Ausbeute** |
| 1e | 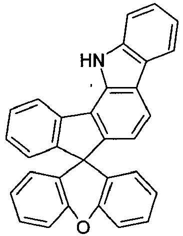 | 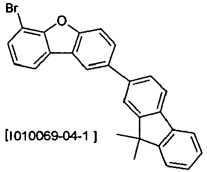 | 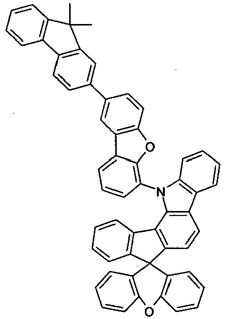 | 39% |
| 2e | 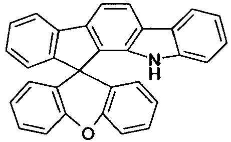 | 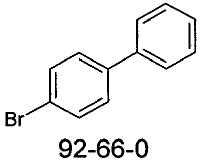 | 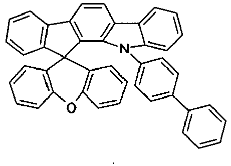 | 41% |
| 3e | 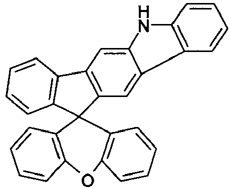 | 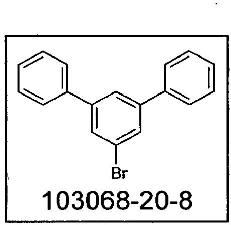 | 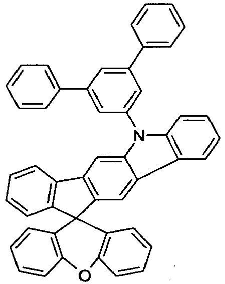 | 42% |
| 4e | 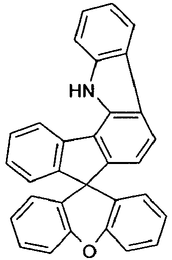 | 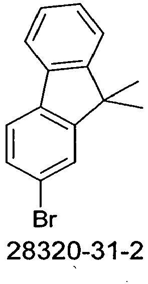 | 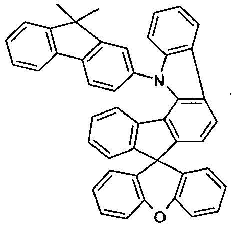 | 34%% |
| 5e | 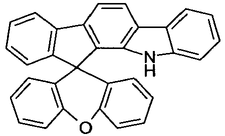 | 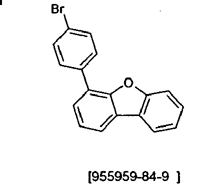 | 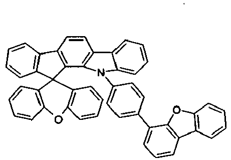 | 45% |
| 6e | 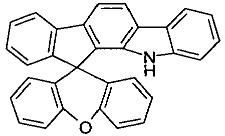 | 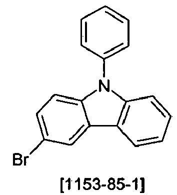 | 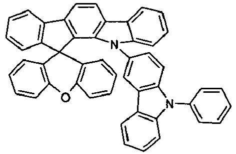 | 42% |
| 7e | 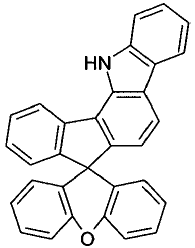 | 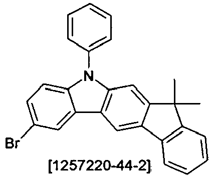 | 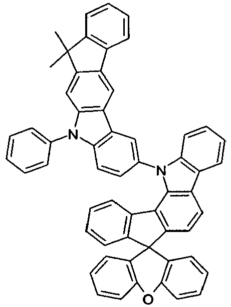 | 40% |
| 8e | 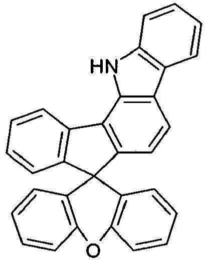 | 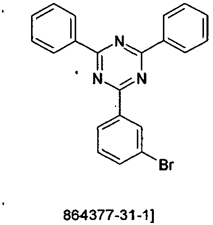 | 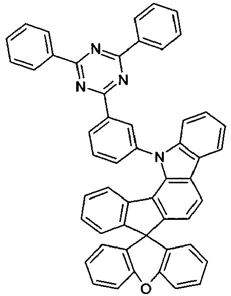 | 47% |

### Beispiel: Herstellung der OLEDs

In den folgenden Beispielen V1 bis E11 (siehe Tabellen 1 und 2) werden die Daten verschiedener OLEDs vorgestellt. Gereinigte Glasplättchen (Reinigung in Laborspülmaschine, Reiniger Merck Extran), die mit strukturiertem ITO (Indium Zinn Oxid) der Dicke 50 nm beschichtet sind, werden 25 min mit UV-Ozon vorbehandelt (UV-Ozon Generator PR-100, Firma UVP) und zur verbesserten Prozessierung mit 20 nm PEDOT:PSS beschichtet (Poly(3,4-ethylendioxythiophen)poly(styrolsulfonat), bezogen als CLEVIOS™ P VP AI 4083 von Heraeus Precious Metals GmbH Deutschland, aus wässriger Lösung aufgeschleudert) und anschließend bei 180°C 10 min lang ausgeheizt. Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Eine Bezeichnung wie "e" oder "6e" in Tabelle 1 bezieht sich auf die entsprechenden in Tabelle 3 gezeigten Materialien. Die weiteren zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrix-material (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC2:6e:TEG1 (40%:50%:10%) bedeutet hierbei, dass das Material IC2 in einem Volumenanteil von 40%, 6e in einem Anteil von 40% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m².

Die Daten der verschiedenen OLEDs sind in Tabelle 2 zusammengefasst. Die Beispiel V1-V5 sind Vergleichsbeispiele gemäß dem Stand der Technik, die Beispiele E1-E11 zeigen Daten von OLEDs mit erfindungsgemäßen Materialien.

Im Folgenden werden einige der Beispiele näher erläutert, um die Vorteile der erfindungsgemäßen Verbindungen zu verdeutlichen.

### Verwendung von erfindungsgemäßen Verbindungen als Elektronentransportmaterialien

Durch den Einsatz von erfindungsgemäßen Verbindungen in der Elektronentransportschicht von OLEDs lassen sich deutliche Steigerungen hinsichtlich Betriebsspannung, externer Quanteneffizienz und damit vor allem auch der Leistungseffizienz erzielen. Siehe hierzu die Beispiele V1, V2 und E1-E3.

### Verwendung von erfindungsgemäßen Verbindungen als Matrixmaterialien in phosphoreszierenden OLEDs

Die erfindungsgemäßen Materialien ergeben bei Einsatz als Matrixmaterialien in phosphoreszierenden OLEDs wesentliche Verbesserungen gegenüber dem Stand der Technik. Mit den Verbindungen 6d, 1d, 8e erhält man beispielsweise deutlich niedrigere Betriebsspannung und höhere Effizienz als mit den Verbindungen StdT1 und StdT2. Siehe hierzu die Beispiele V3, V4 und E9-E11.

Weiterhin lassen sich durch erfindungsgemäße Verbindungen Verbesserungen bei Mischung mit einem zweiten Matrixmaterial erzielen. Gegenüber der Verbindung StdT3, die in Kombination mit IC2 bereits sehr gute Leistungsdaten liefert, erhält man eine Verbesserung durch den Einsatz der Verbindungen e und 6e. Siehe hierzu die Beispiele V5, E5 und E6.

**Tabelle 1: Aufbau der OLEDs**

| Bsp. | HTL | IL | EBL | EML | HBL | ETL | EIL |
|---|---|---|---|---|---|---|---|
| | Dicke | Dicke | Dicke | Dicke | Dicke | Dicke | Dicke |
| V1 | SpA1 | HATCN | SpMA1 | IC1:TEG1 | --- | StdT1 | LiQ |
| | 70nm | 5nm | 90nm | (90%:10%) 30nm | | 40nm | 4nm |
| V2 | SpA1 | HATCN | SpMA1 | IC1:TEG1 | --- | StdT2 | LiQ |
| | 70nm | 5nm | 90nm | (90%:10%) 30nm | | 40nm | 4nm |
| V3 | SpA1 | HATCN | SpMA1 | StdT1:TEG1 (90%:10%) | --- | ST2:LiQ | --- |
| | 70nm | 5nm | 90nm | 30nm | | (50%:50%) 40nm | |
| V4 | SpA1 | HATCN | SpMA1 | StdT2:TEG1 (90%:10%) | IC1 | ST2:LiQ | --- |
| | 70nm | 5nm | 90nm | 30nm | 10nm | (50%:50%) 30nm | |
| V5 | SpA1 | HATCN | SpMA1 | IC2:StdT3:TEG1 | IC1 | ST2:LiQ | --- |
| | 70nm | 5nm | 90nm | (40%:50%:10%) 30nm | 10nm | (50%:50%) 30nm | |
| E1 | SpA1 | HATCN | SpMA1 | IC1:TEG1 | --- | d | LiQ |
| | 70nm | 5nm | 90nm | (90%:10%) 30nm | | 40nm | 4nm |
| E2 | SpA1 | HATCN | SpMA1 | IC1:TEG1 | - | 9d | LiQ |
| | 70nm | 5nm | 90nm | (90%:10%) 30nm | | 40nm | 4nm |
| E3 | SpA1 | HATCN | SpMA1 | IC1:TEG1 | --- | 8e | LiQ |
| | 70nm | 5nm | 90nm | (90%:10%) 30nm | | 40nm | 4nm |
| E4 | SpA1 | HATCN | SpMA1 | d:TER1 | --- | ST2:LiQ | --- |
| | 90nm | 5nm | 130nm | (92%:8%) 40nm | | (50%:50%) 40nm | |
| E5 | SpA1 | HATCN | SpMA1 | IC2:e:TEG1 | IC1 | ST2:LiQ | --- |
| | 70nm | 5nm | 90nm | (40%:50%:10%) 30nm | 10nm | (50%:50%) 30nm | |
| E6 | SpA1 | HATCN | SpMA1 | IC2:6e:TEG1 | IC1 | ST2:LiQ | --- |
| | 70nm | 5nm | 90nm | (40%:50%:10%) 30nm | 10nm | (50%:50%) 30nm | |
| E7 | SpA1 | HATCN | SpMA1:5e | IC1:TEG1 | IC1 | ST2:LiQ | --- |
| | 70nm | 5nm | (85%:15%) 90nm | (90%:10%) 30nm | 10nm | (50%:50%) 30nm | |
| ES | SpA1 | HATCN | SpMA1 | 10d:BIC1:TEG1 | IC1 | ST2:LiQ | --- |
| | 70nm | 5nm | 90nm | (55%:40%:5%) 30nm | 10nm | (50%:50%) 30nm | |
| E9 | SpA1 | HATCN | SpMA1 | 6d:TEG1 (90%:10%) | --- | ST2:LiQ | --- |
| | 70nm | 5nm | 90nm | 30nm | | (50%:50%) 40nm | |
| E10 | SpA1 | HATCN | SpMA1 | 1d:TEG1 (90%:10%) | --- | ST2:LiQ | --- |
| | 70nm | 5nm | 90nm | 30nm | | (50%:50%) 40nm | |
| | | | | | | | |
| E11 | SpA1 | HATCN | SpMA1 | 8e:TEG1 (90%:10%) | IC1 | ST2:LiQ | --- |
| | 70nm | 5nm | 90nm | 30nm | 10nm | (50%:50%) 30nm | |

**Tabelle 2: Daten der OLEDs**

| Bsp. | U1000 (V) | SE1000 (cd/A) | LE1000 (Im/W) | EQE 1000 | CIE x/y bei 1000 cd/m² |
|---|---|---|---|---|---|
| V1 | 3.6 | 51 | 45 | 14.5% | 0.34/0.62 |
| V2 | 3.8 | 54 | 44 | 15.2% | 0.33/0.62 |
| V3 | 3.5 | 51 | 46 | 14.3% | 0.33/0.62 |
| V4 | 3.7 | 56 | 48 | 15.7% | 0.33/0.62 |
| V5 | 3.3 | 59 | 55 | 16.2% | 0.33/0.62 |
| E1 | 3.0 | 58 | 61 | 16.3% | 0.34/0.62 |
| E2 | 3.4 | 56 | 52 | 15.6% | 0.33/0.63 |
| E3 | 3.2 | 60 | 59 | 17.0% | 0.33/0.62 |
| E4 | 4.1 | 12.1 | 9.4 | 13.1% | 0.67/0.33 |
| E5 | 3.2 | 61 | 59 | 17.2% | 0.34/0.61 |
| E6 | 3.3 | 65 | 63 | 18.2% | 0.34/0.62 |
| E7 | 3.6 | 59 | 52 | 16.7% | 0.34/0.62 |
| E8 | 3.3 | 60 | 57 | 16.6% | 0.33/0.62 |
| E9 | 3.4 | 67 | 61 | 18.6% | 0.33/0.62 |
| E10 | 3.2 | 61 | 60 | 17.1% | 0.34/0.62 |
| E11 | 3.2 | 59 | 57 | 16.5% | 0.33/0.62 |

**Tabelle 2: Strukturformeln der Materialien für die OLEDs**

| | |
|---|---|
| 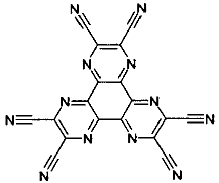 | 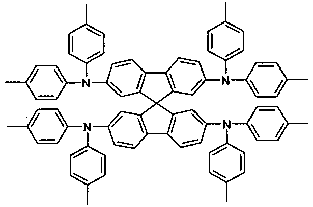 |
| HATCN | SpA1 |
| 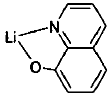 | 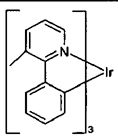 |
| LiQ | TEG1 |
| 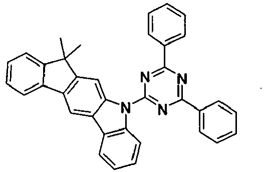 | 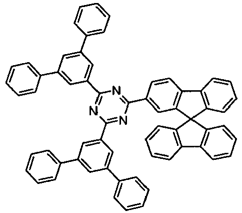 |
| IC1 | ST2 |
| 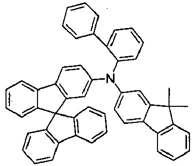 | 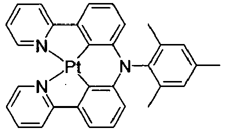 |
| SpMA1 | TER1 |
| 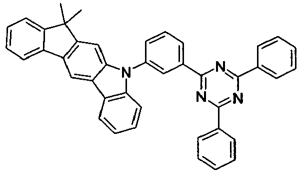 | 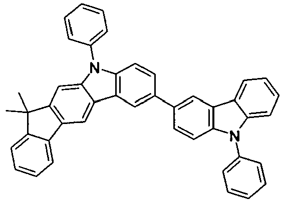 |
| IC2 | BIC1 |
| 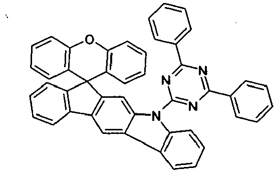 | 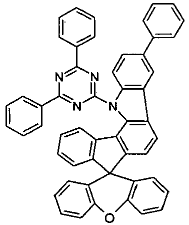 |
| d | 1d |
| 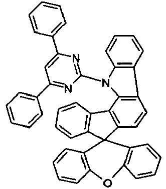 | 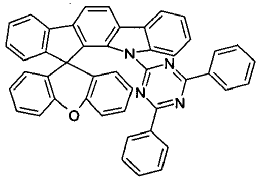 |
| 6d | 9d |
| 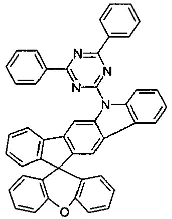 | 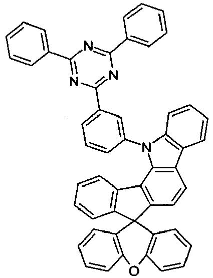 |
| 10d | 8e |
| 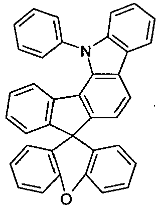 | 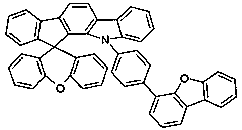 |
| e | 5e |
| 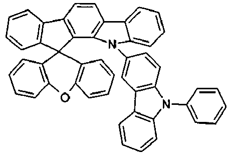 | 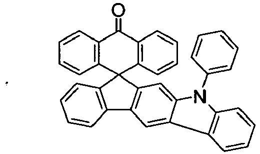 |
| 6e | StdT1 |
| 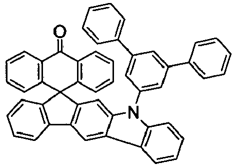 | 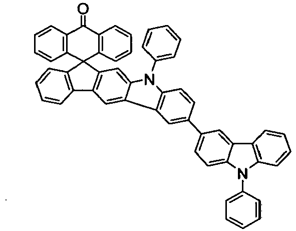 |
| StdT2 | StdT2 |

## Patentansprüche

1. Verbindung gemäß Formel (1), 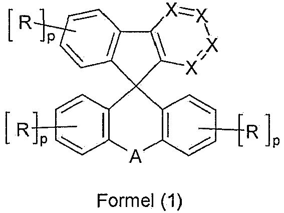 wobei für die verwendeten Symbole und Indizes gilt:
A ist O oder S;
**dadurch gekennzeichnet, dass** für die Verbindung der Formel (1) gilt:
X zwei benachbarte X stehen für eine Gruppe der Formel (2), 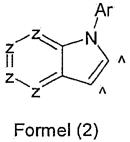 wobei ^ die entsprechenden benachbarten Gruppen X in Formel (1) andeutet, und die verbleibenden beiden Gruppen X stehen für CR;
Z steht für CR; oder zwei benachbarte Z stehen für eine Gruppe der Formel (2a) und die anderen beiden Z stehen für CR, 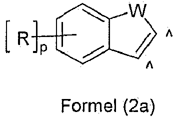 wobei ^ die entsprechenden benachbarten Gruppen Z in Formel (2) andeutet; dabei steht W für O, S, NR oder CR₂;
Ar ist ein aromatisches oder heteroaromatisches Ringsystem mit 5 bis 40 aromatischen Ringatomen, das mit einem oder mehreren Resten R substituiert sein kann;
R ist bei jedem Auftreten gleich oder verschieden ausgewählt aus der Gruppe bestehend aus H, D, F, Cl, Br, I, CN, NO₂, N(Ar¹)₂, N(R¹)₂, C(=O)Ar¹, C(=O)R¹, P(=O)(Ar¹)₂, P(Ar¹)₂, B(Ar¹)₂, Si(Ar¹)₃, Si(R¹)₃, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R¹ substituiert sein kann, wobei eine oder mehrere nicht-benachbarte CH₂-Gruppen durch R¹C=CR¹, C≡C, Si(R¹)₂, C=O, C=S, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R¹ substituiert sein kann, einer Aryloxy- oder Heteroaryloxygruppe mit 5 bis 40 aromatischen Ringatomen, die mit einem oder mehreren Resten R¹ substituiert sein kann; dabei können optional zwei benachbarte Substituenten R ein monocyclisches oder polycyclisches, aliphatisches, aromatisches oder heteroaromatisches Ringsystem bilden, das mit einem oder mehreren Resten R¹ substituiert sein kann;
Ar¹ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R¹ substituiert sein kann; dabei können zwei Reste Ar¹, welche an dasselbe N-, P-, B- oder Si-Atom binden, auch durch eine Einfachbindung oder eine Brücke, ausgewählt aus N(R¹), C(R¹)₂, O oder S, miteinander verbrückt sein;
R¹ ist bei jedem Auftreten gleich oder verschieden ausgewählt aus der Gruppe bestehend aus H, D, F, CN, einem aliphatischen Kohlenwasserstoffrest mit 1 bis 20 C-Atomen, oder einem aromatischen oder heteroaromatischen Ringsystem mit 5 bis 30 aromatischen Ringatomen, in dem ein oder mehrere H-Atome durch D, F, CN oder eine Alkylgruppe mit 1 bis 10 C-Atomen ersetzt sein können; dabei können wobei zwei oder mehr benachbarte Substituenten R¹ miteinander ein mono- oder polycyclisches, aliphatisches Ringsystem bilden;
p ist bei jedem Auftreten gleich oder verschieden 0, 1, 2, 3 oder 4.

2. Verbindung nach Anspruch 1 ausgewählt aus den Verbindungen der Formeln (3) bis (8), 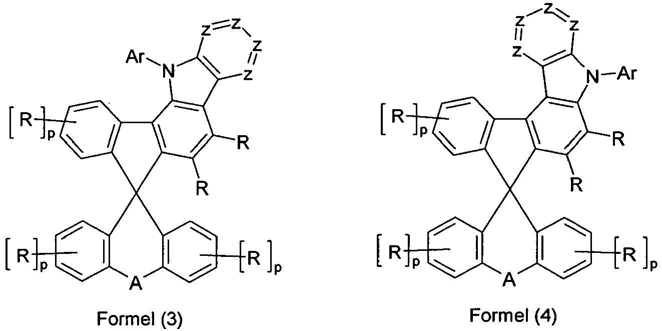 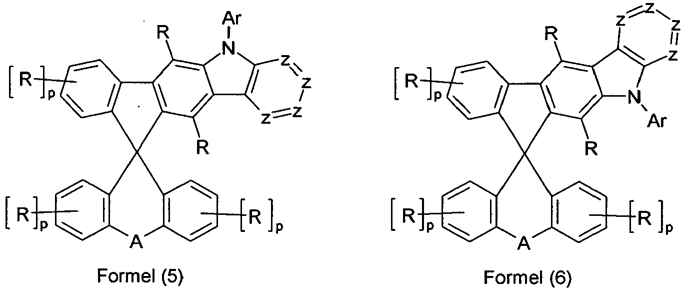 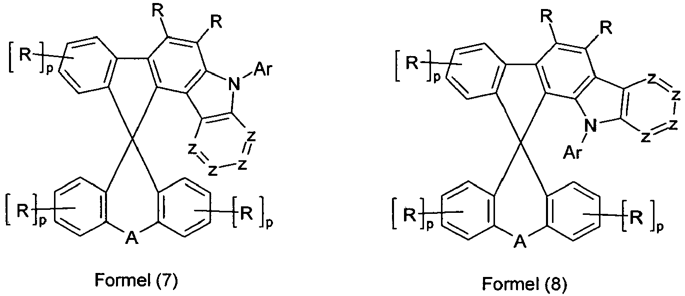 wobei die verwendeten Symbole und Indizes die in Anspruch 1 genannten Bedeutungen aufweisen.

3. Verbindung nach Anspruch 1 oder 2, **dadurch gekennzeichnet, dass** p bei jedem Auftreten gleich oder verschieden 0 oder 1 ist.

4. Verbindung nach einem oder mehreren der Ansprüche 1 bis 3, ausgewählt aus den Verbindungen der Formeln (3a) bis (8a), 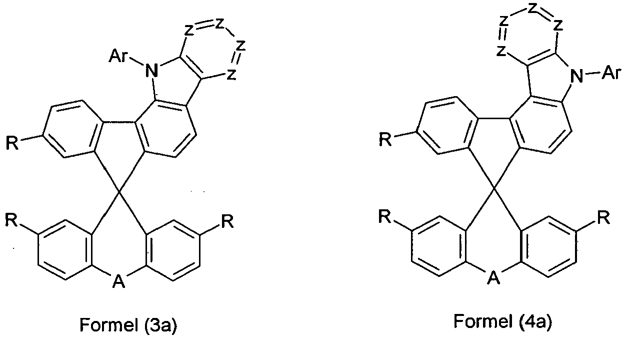 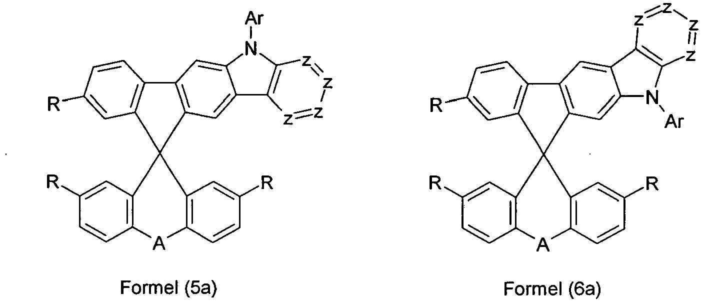 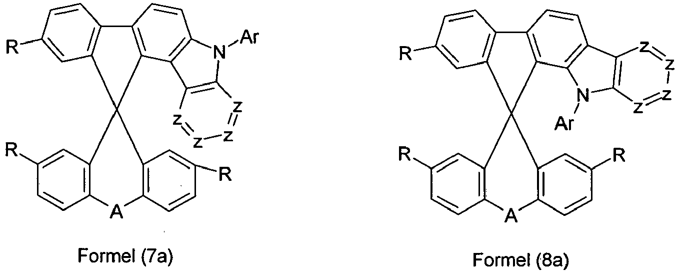 wobei die verwendeten Symbole die in Anspruch 1 genannten Bedeutungen aufweisen.

5. Verbindung nach einem oder mehreren der Ansprüche 1 bis 4, ausgewählt aus den Verbindungen der Formeln (3b) bis (8b), 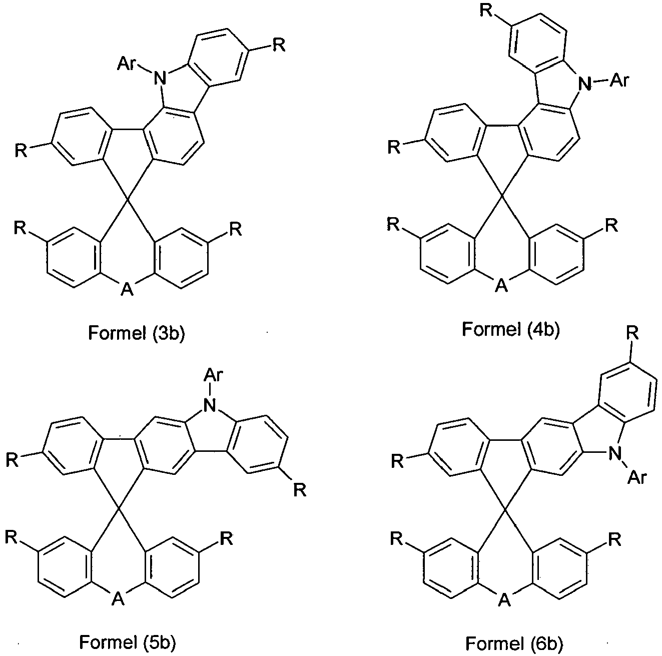 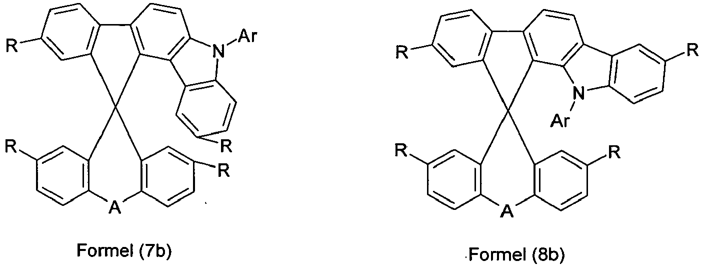 wobei die verwendeten Symbole die in Anspruch 1 genannten Bedeutungen aufweisen.

6. Verbindung nach einem oder mehreren der Ansprüche 1 bis 5, **dadurch gekennzeichnet, dass** A für Sauerstoff steht.

7. Verbindung nach einem oder mehreren der Ansprüche 1 bis 6, ausgewählt aus den Verbindungen der Formeln (3d) bis (8d), 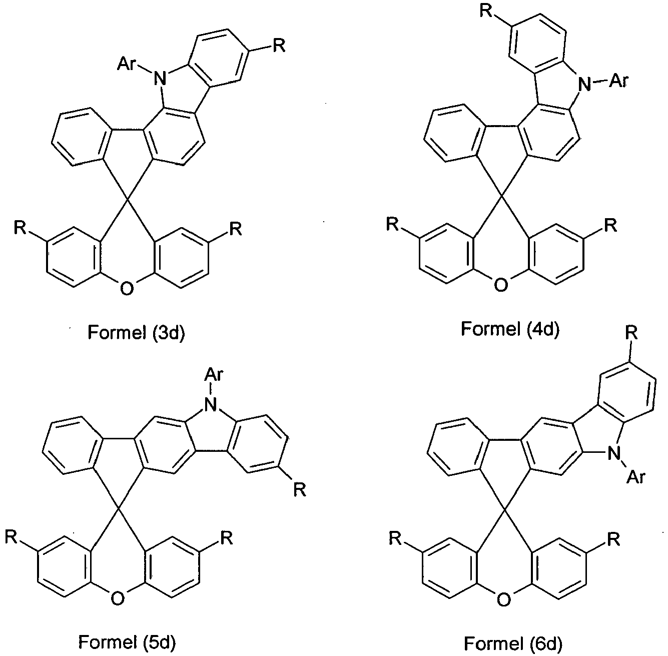 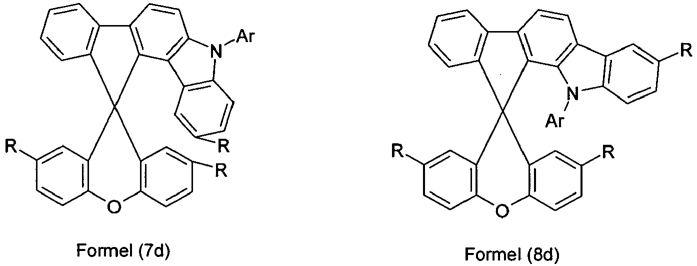 wobei die verwendeten Symbole die in Anspruch 1 genannten Bedeutungen aufweisen.

8. Verbindung nach einem oder mehreren der Ansprüche 1 bis 7, **dadurch gekennzeichnet, dass** Ar ausgewählt ist aus der Gruppe bestehend aus Phenyl, Biphenyl, Terphenyl, Quaterphenyl, Fluoren, Spirobifluoren, Naphthalin, Indol, Benzofuran, Benzothiophen, Carbazol, Dibenzofuran, Dibenzothiophen, Indenocarbazol, Indolocarbazol, Pyridin, Pyrimidin, Pyrazin, Pyridazin, Triazin, Phenanthren, Triphenylen oder Kombinationen aus zwei oder drei dieser Gruppen, wobei diese Gruppen jeweils mit einem oder mehreren Resten R substituiert sein können.

9. Verbindung nach einem oder mehreren der Ansprüche 1 bis 8, **dadurch gekennzeichnet, dass** R gleich oder verschieden bei jedem Auftreten ausgewählt ist aus der Gruppe bestehend aus H, D, F, CN, N(Ar¹)₂, C(=O)Ar¹, P(=O)(Ar¹)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R¹ substituiert sein kann, wobei eine oder mehrere nichtbenachbarte CH₂-Gruppen durch O ersetzt sein können und wobei ein oder mehrere H-Atome durch D oder F ersetzt sein können, oder einem aromatischen oder heteroaromatischen Ringsystem mit 5 bis 30 aromatischen Ringatomen, das jeweils mit einem oder mehreren Resten R¹ substituiert sein kann.

10. Verfahren zur Herstellung einer Verbindung nach einem oder mehreren der Ansprüche 1 bis 9, umfassend die Reaktionsschritte:
a) Synthese der Grundgerüsts, die noch keine Gruppe Ar enthält; und
b) Umsetzung der Grundgerüsts aus a) in einer C-N-Kupplung oder in einer nukleophilen aromatischen Substitutionsreaktion zur Einführung der Gruppe Ar.

11. Formulierung, enthaltend mindestens eine Verbindung nach einem oder mehreren der Ansprüche 1 bis 9 und mindestens ein Lösemittel.

12. Verwendung einer Verbindung nach einem oder mehreren der Ansprüche 1 bis 9 in einer elektronischen Vorrichtung oder einer Formulierung nach Anspruch 11 zur Herstellung einer elektronischen Vorrichtung.

13. Elektronische Vorrichtung enthaltend mindestens eine Verbindung nach einem oder mehreren der Ansprüche 1 bis 9.

14. Elektronische Vorrichtung nach Anspruch 13, wobei es sich um einer organische Elektrolumineszenzvorrichtung handelt, **dadurch gekennzeichnet, dass** die Verbindung nach einem oder mehreren der Ansprüche 1 bis 9 in einer emittierenden Schicht als Matrixmaterial für phosphoreszierende Emitter oder in einer Elektronentransportschicht oder in einer Lochtransportschicht oder in einer Exzitonenblockierschicht oder in einer Lochblockierschicht eingesetzt wird.

## Claims

1. Compound of formula (1) 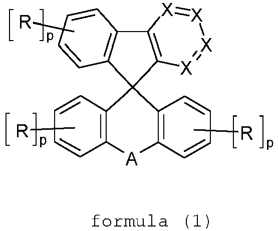 where the symbols and indices used are as follows:
A is O or S;
**characterized in that**, for the compound of the formula (1):
X two adjacent X are a group of the formula (2) 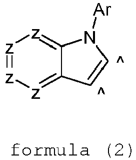
where ^ indicates the corresponding adjacent X groups in formula (1), and the two remaining X groups are CR;
Z is CR; or two adjacent Z are a group of the formula (2a) and the two other Z are CR, 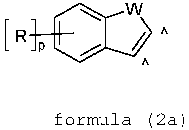
where ^ indicates the corresponding adjacent Z groups in formula (2); W here is O, S, NR or CR₂;
Ar is an aromatic or heteroaromatic ring system which has 5 to 40 aromatic ring atoms and may be substituted by one or more R radicals;
R is the same or different at each instance and is selected from the group consisting of H, D, F, Cl, Br, I, CN, NO₂, N(Ar¹)₂, N(R¹)₂, C(=O)Ar¹, C(=O)R¹, P(=O)(Ar¹)₂, P(Ar¹)₂, B(Ar¹)₂, Si(Ar¹)₃, Si(R¹)₃, a straight-chain alkyl, alkoxy or thioalkyl group having 1 to 40 carbon atoms or a branched or cyclic alkyl, alkoxy or thioalkyl group having 3 to 40 carbon atoms or an alkenyl or alkynyl group having 2 to 40 carbon atoms, each of which may be substituted by one or more R¹ radicals, where one or more nonadjacent CH₂ groups may be replaced by R¹C=CR¹, C≡C, Si(R¹)₂, C=O, C=S, C=NR¹, P(=O)(R¹), SO, SO₂, NR¹, O, S or CONR¹ and where one or more hydrogen atoms may be replaced by D, F, Cl, Br, I, CN or NO₂, an aromatic or heteroaromatic ring system which has 5 to 60 aromatic ring atoms and may be substituted in each case by one or more R¹ radicals, an aryloxy or heteroaryloxy group which has 5 to 40 aromatic ring atoms and may be substituted by one or more R¹ radicals; at the same time, it is optionally possible for two adjacent R substituents to form a monocyclic or polycyclic, aliphatic, aromatic or heteroaromatic ring system which may be substituted by one or more R¹ radicals;
Ar¹ is the same or different at each instance and is an aromatic or heteroaromatic ring system which has 5 to 30 aromatic ring atoms and may be substituted by one or more nonaromatic R¹ radicals; at the same time, two Ar¹ radicals bonded to the same nitrogen, phosphorus, boron or silicon atom may also be bridged to one another by a single bond or a bridge selected from N(R¹), C(R¹)₂, O and S;
R¹ is the same or different at each instance and is selected from the group consisting of H, D, F, CN, an aliphatic hydrocarbyl radical having 1 to 20 carbon atoms, or an aromatic or heteroaromatic ring system having 5 to 30 aromatic ring atoms in which one or more hydrogen atoms may be replaced by D, F, CN or an alkyl group having 1 to 10 carbon atoms; at the same time, two or more adjacent R¹ substituents together may form a mono- or polycyclic, aliphatic ring system;
p is the same or different at each instance and is 0, 1, 2, 3 or 4.

2. Compound according to Claim 1, selected from the compounds of the formulae (3) to (8) 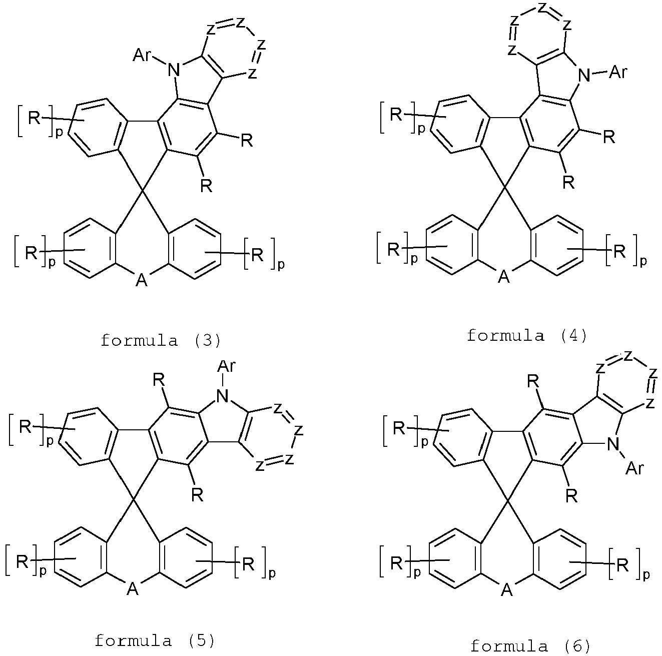 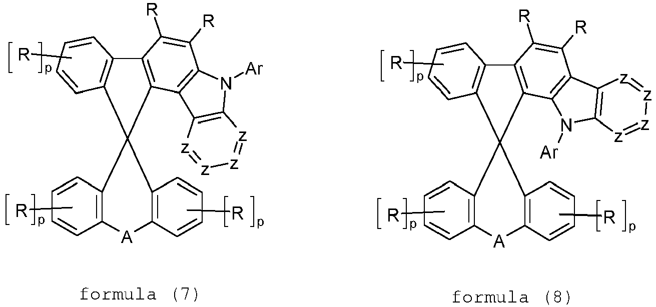 where the symbols and indices used have the definitions given in Claim 1.

3. Compound according to Claim 1 or 2, **characterized in that** p is the same or different at each instance and is 0 or 1.

4. Compound according to one or more of Claims 1 to 3, selected from the compounds of the formulae (3a) to (8a) 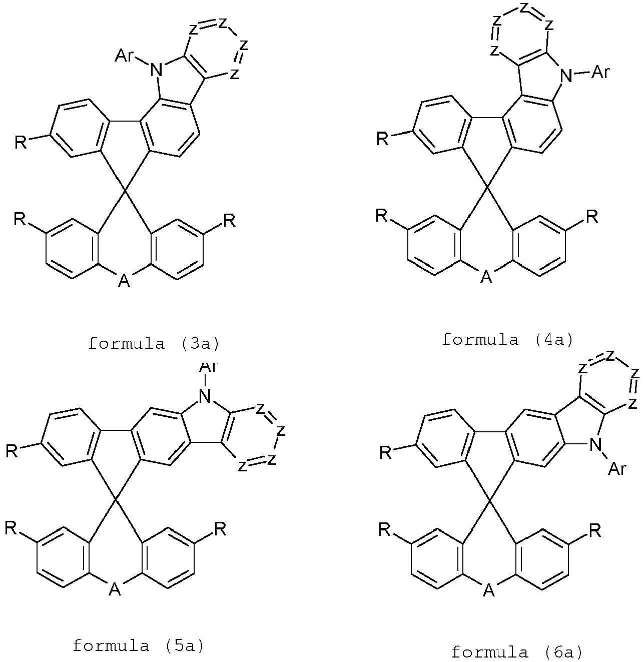 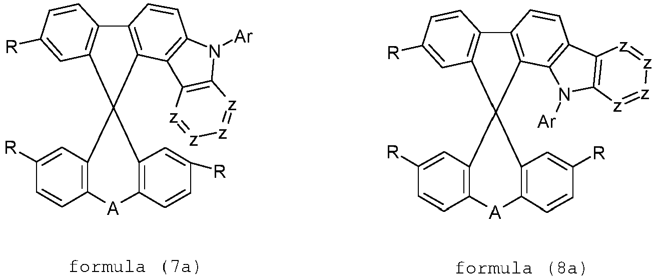 where the symbols used have the definitions given in Claim 1.

5. Compound according to one or more of Claims 1 to 4, selected from the compounds of the formulae (3b) to (8b) 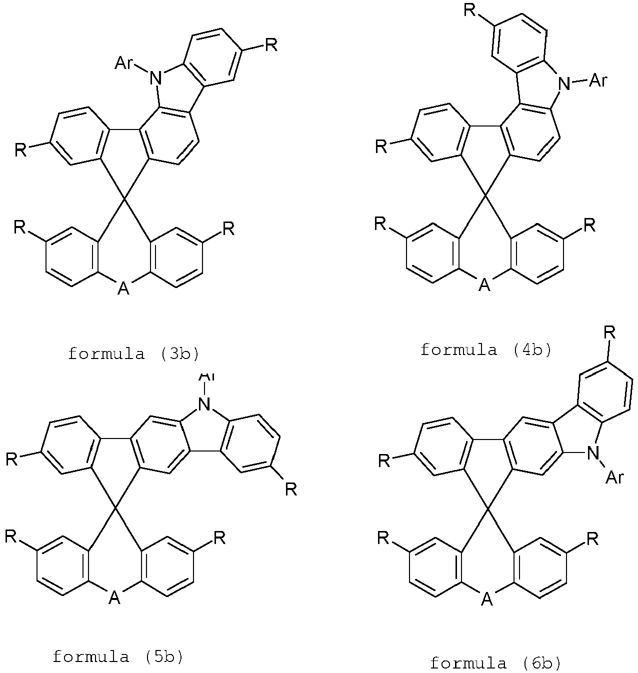 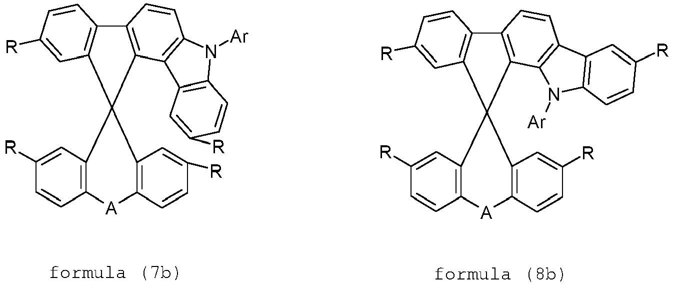 where the symbols used have the definitions given in Claim 1.

6. Compound according to one or more of Claims 1 to 5, **characterized in that** A is oxygen.

7. Compound according to one or more of Claims 1 to 6, selected from the compounds of the formulae (3d) to (8d) 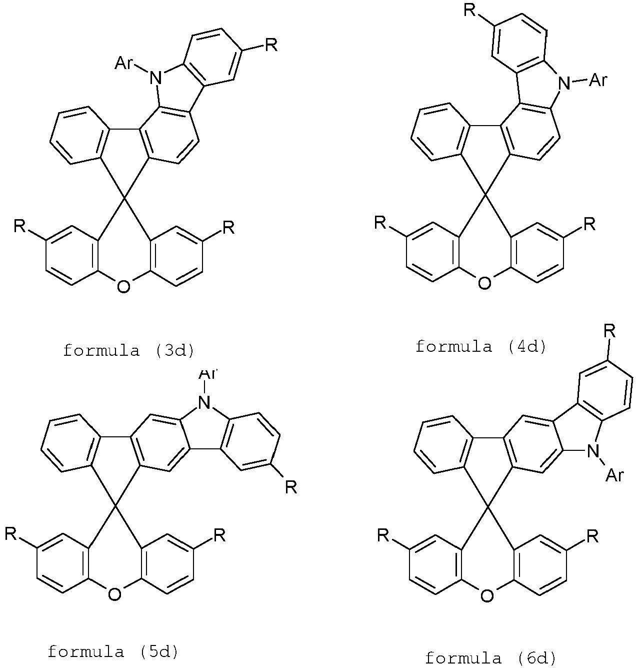 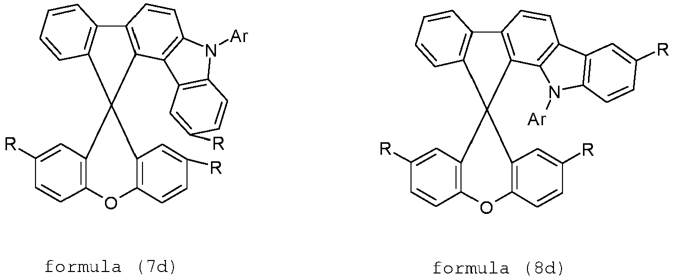 where the symbols used have the definitions given in Claim 1.

8. Compound according to one or more of Claims 1 to 7, **characterized in that** Ar is selected from the group consisting of phenyl, biphenyl, terphenyl, quaterphenyl, fluorene, spirobifluorene, naphthalene, indole, benzofuran, benzothiophene, carbazole, dibenzofuran, dibenzothiophene, indenocarbazole, indolocarbazole, pyridine, pyrimidine, pyrazine, pyridazine, triazine, phenanthrene, triphenylene or combinations of two or three of these groups, where these groups may each be substituted by one or more R radicals.

9. Compound according to one or more of Claims 1 to 8, **characterized in that** R is the same or different at each instance and is selected from the group consisting of H, D, F, CN, N(Ar¹)₂, C(=O)Ar¹, P(=O)(Ar¹)₂, a straight-chain alkyl or alkoxy group having 1 to 10 carbon atoms or a branched or cyclic alkyl or alkoxy group having 3 to 10 carbon atoms or an alkenyl group having 2 to 10 carbon atoms, each of which may be substituted by one or more R¹ radicals, where one or more nonadjacent CH₂ groups may be replaced by O and where one or more hydrogen atoms may be replaced by D or F, or an aromatic or heteroaromatic ring system which has 5 to 30 aromatic ring atoms and may be substituted in each case by one or more R¹ radicals.

10. Process for preparing a compound according to one or more of Claims 1 to 9, comprising the reaction steps of:
a) synthesizing the base skeleton which does not yet contain an Ar group; and
b) converting the base skeleton from a) in a C-N coupling or in a nucleophilic aromatic substitution reaction for introduction of the Ar group.

11. Formulation comprising at least one compound according to one or more of Claims 1 to 9 and at least one solvent.

12. Use of a compound according to one or more of Claims 1 to 9 in an electronic device or of a formulation according to Claim 11 for production of an electronic device.

13. Electronic device comprising at least one compound according to one or more of Claims 1 to 9.

14. Electronic device according to Claim 13 which is an organic electroluminescent device, **characterized in that** the compound according to one or more of Claims 1 to 9 is used in an emitting layer as matrix material for phosphorescent emitters or in an electron transport layer or in a hole transport layer or in an exciton blocker layer or in a hole blocker layer.

## Revendications

1. Composé selon la formule (1), 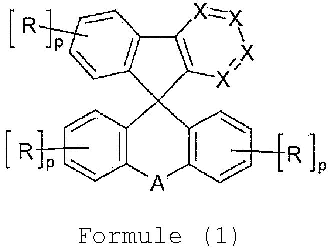 dans laquelle, pour les symboles et indices utilisés :
A représente O ou S ;
**caractérisé en ce que**, pour le composé de la formule (1) :
X deux X voisins représentent un groupe de la formule (2), 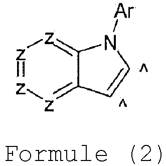
dans laquelle ^ indique les groupes X voisins correspondants dans la formule (1), et les deux groupes X restants représentent CR ;
Z représente CR ; ou deux Z voisins représentent un groupe de la formule (2a) et les deux autres Z représentent CR, 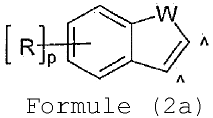 dans laquelle ^ indique les groupes Z voisins correspondants dans la formule (2) ; W représente O, S, NR ou CR₂ ;
Ar représente un système cyclique aromatique ou hétéroaromatique de 5 à 40 atomes de cycle aromatique, qui peut être substitué avec un ou plusieurs radicaux R ;
les R sont choisis à chaque occurrence de manière identique ou différente dans le groupe constitué par H, D, F, Cl, Br, I, CN, NO₂, N(Ar¹)₂, N(R¹)₂, C(=O)Ar¹, C(=O)R¹, P(=O)(Ar¹)₂, P(Ar¹)₂, B(Ar¹)₂, Si(Ar¹)₃, Si(R¹)₃, un groupe alkyle, alcoxy ou thioalkyle linéaire de 1 à 40 atomes C ou un groupe alkyle, alcoxy ou thioalkyle ramifié ou cyclique de 3 à 40 atomes C ou un groupe alcényle ou alcynyle de 2 à 40 atomes C, qui peut respectivement être substitué avec un ou plusieurs radicaux R¹, un ou plusieurs groupes CH₂ non voisins pouvant être remplacés par R¹C=CR¹, C≡C, Si(R¹)₂, C=O, C=S, C=NR¹, P(=O)(R¹), SO, SO₂, NR¹, O, S ou CONR¹, et un ou plusieurs atomes H pouvant être remplacés par D, F, Cl, Br, I, CN ou NO₂, un système cyclique aromatique ou hétéroaromatique de 5 à 60 atomes de cycle aromatique, qui peut respectivement être substitué avec un ou plusieurs radicaux R¹, un groupe aryloxy ou hétéroaryloxy de 5 à 40 atomes de cycle aromatique, qui peut être substitué avec un ou plusieurs radicaux R¹ ; deux substituants R voisins pouvant éventuellement former un système cyclique monocyclique ou polycyclique, aliphatique, aromatique ou hétéroaromatique, qui peut être substitué avec un ou plusieurs radicaux R¹ ;
les Ar¹ représentent à chaque occurrence de manière identique ou différente un système cyclique aromatique ou hétéroaromatique de 5 à 30 atomes de cycle aromatique, qui peut être substitué avec un ou plusieurs radicaux non aromatiques R¹ ; deux radicaux Ar¹, qui sont reliés au même atome N, P, B ou Si, pouvant également être pontés l'un avec l'autre par une simple liaison ou un pont, choisi parmi N(R¹), C(R¹)₂, O ou S ;
les R¹ sont choisis à chaque occurrence de manière identique ou différente dans le groupe constitué par H, D, F, CN, un radical hydrocarboné aliphatique de 1 à 20 atomes C, ou un système cyclique aromatique ou hétéroaromatique de 5 à 30 atomes de cycle aromatiques, dans lequel un ou plusieurs atomes H peuvent être remplacés par D, F, CN ou un groupe alkyle de 1 à 10 atomes C ; deux ou davantage de substituants R¹ voisins pouvant former les uns avec les autres un système cyclique aliphatique mono- ou polycyclique ; les p représentent à chaque occurrence de manière identique ou différente 0, 1, 2, 3 ou 4.

2. Composé selon la revendication 1 choisi parmi les composés des formules (3) à (8), 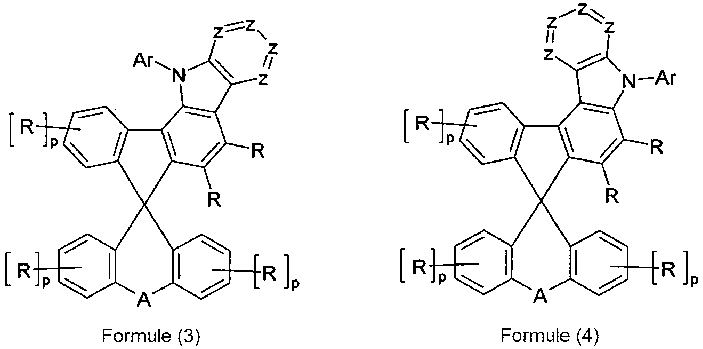 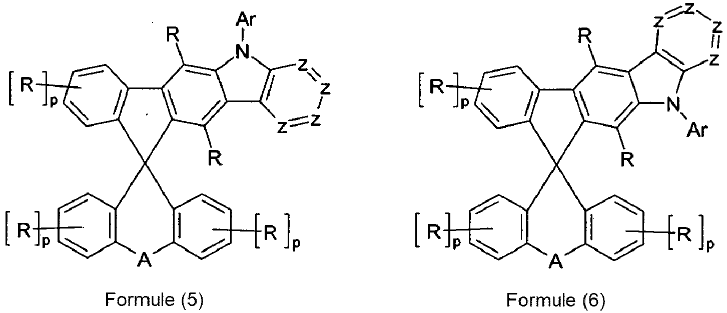 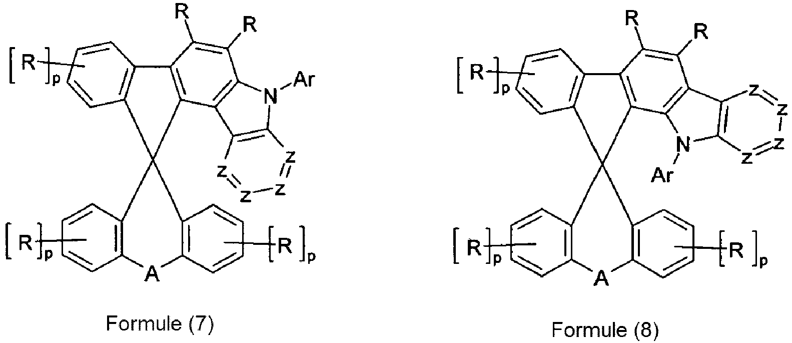 dans lesquelles les symboles et indices utilisés ont les significations indiquées dans la revendication 1.

3. Composé selon la revendication 1 ou 2, **caractérisé en ce que** les p représentent à chaque occurrence de manière identique ou différente 0 ou 1.

4. Composé selon une ou plusieurs des revendications 1 à 3, choisi parmi les composés des formules (3a) à (8a), 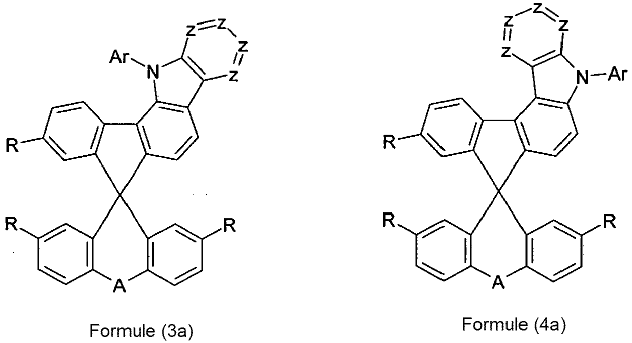 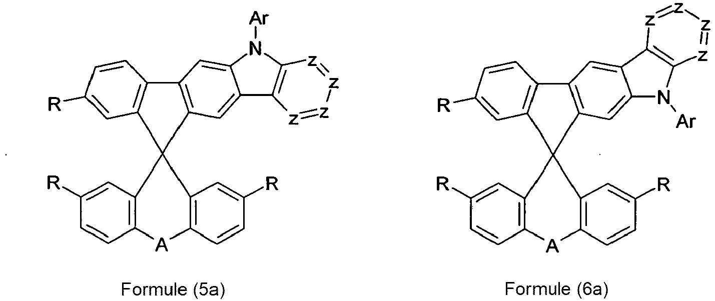 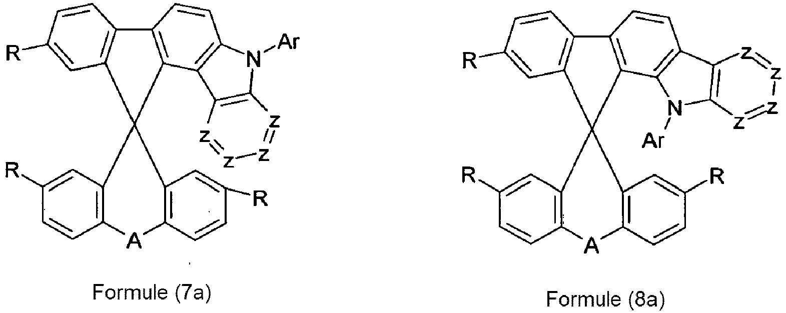 dans lesquelles les symboles utilisés ont les significations indiquées dans la revendication 1.

5. Composé selon une ou plusieurs des revendications 1 à 4, choisi parmi les composés des formules (3b) à (8b), 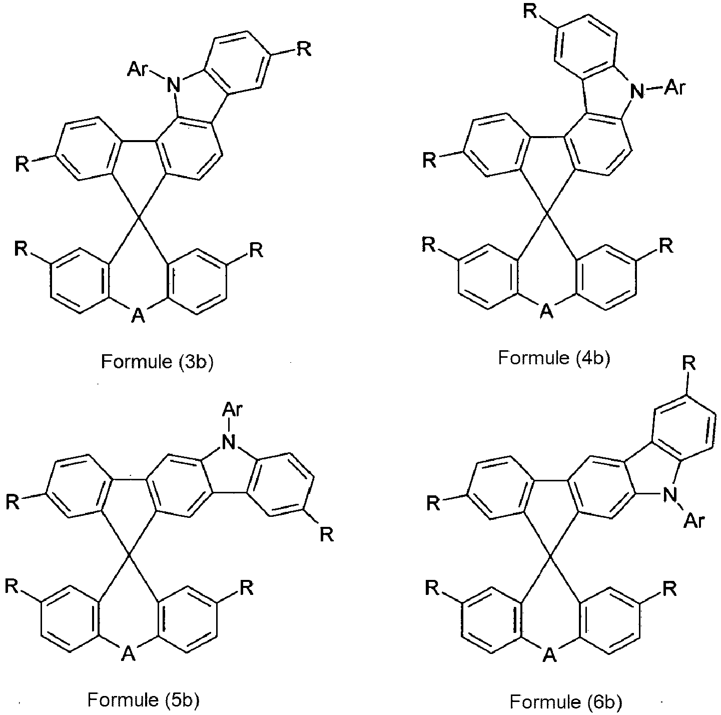 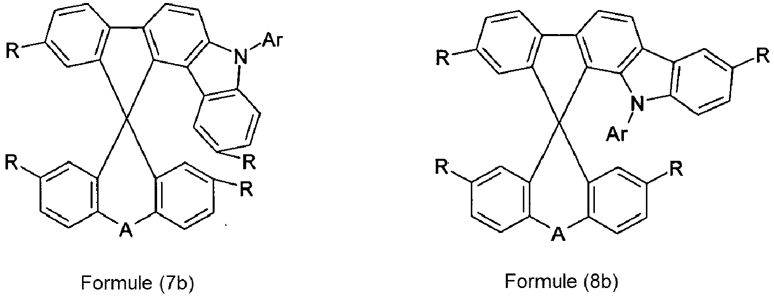 dans lesquelles les symboles utilisés ont les significations indiquées dans la revendication 1.

6. Composé selon une ou plusieurs des revendications 1 à 5, **caractérisé en ce qu'**A représente l'oxygène.

7. Composé selon une ou plusieurs des revendications 1 à 6, choisi parmi les composés des formules (3d) à (8d), 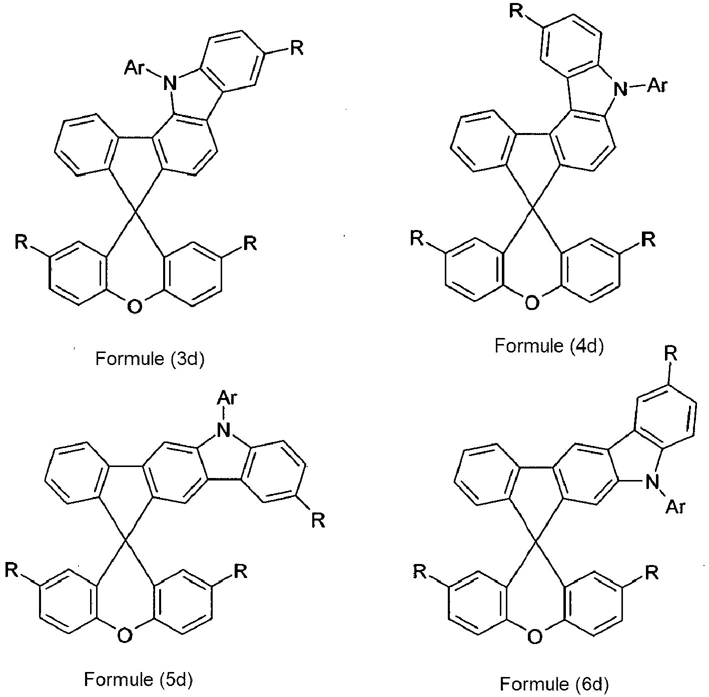 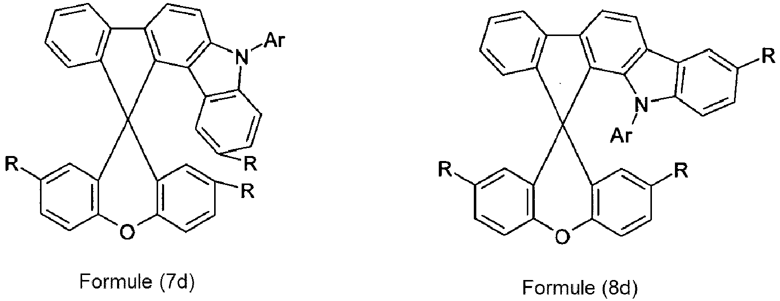 dans lesquelles les symboles utilisés ont les significations indiquées dans la revendication 1.

8. Composé selon une ou plusieurs des revendications 1 à 7, **caractérisé en ce qu'**Ar est choisi dans le groupe constitué par phényle, biphényle, terphényle, quaterphényle, fluorène, spirobifluorène, naphtaline, indène, benzofurane, benzothiophène, carbazole, dibenzofurane, dibenzothiophène, indénocarbazole, indolocarbazole, pyridine, pyrimidine, pyrazine, pyridazine, triazine, phénanthrène, triphénylène ou des combinaisons de deux ou trois de ces groupes, ces groupes pouvant chacun être substitués avec un ou plusieurs radicaux R.

9. Composé selon une ou plusieurs des revendications 1 à 8, **caractérisé en ce que** les R sont choisis de manière identique ou différente à chaque occurrence dans le groupe constitué par H, D, F, CN, N(Ar¹)₂, C(=O)Ar¹, P(=O)(Ar¹)₂, un groupe alkyle ou alcoxy linéaire de 1 à 10 atomes C ou un groupe alkyle ou alcoxy ramifié ou cyclique de 3 à 10 atomes C ou un groupe alcényle de 2 à 10 atomes C, qui peut respectivement être substitué avec un ou plusieurs radicaux R¹, un ou plusieurs groupes CH₂ non voisins pouvant être remplacés par O et un ou plusieurs atomes H pouvant être remplacés par D ou F, ou un système cyclique aromatique ou hétéroaromatique de 5 à 30 atomes de cycle aromatique, qui peut respectivement être substitué avec un ou plusieurs radicaux R¹.

10. Procédé de fabrication d'un composé selon une ou plusieurs des revendications 1 à 9, comprenant les étapes de réaction suivantes :
a) la synthèse d'un squelette de base, qui ne contient pas encore de groupe Ar ; et
b) la mise en réaction du squelette de base de a) dans un couplage C-N ou dans une réaction de substitution aromatique nucléophile pour l'insertion du groupe Ar.

11. Formulation, contenant au moins un composé selon une ou plusieurs des revendications 1 à 9 et au moins un solvant.

12. Utilisation d'un composé selon une ou plusieurs des revendications 1 à 9 dans un dispositif électronique ou d'une formulation selon la revendication 11 pour la fabrication d'un dispositif électronique.

13. Dispositif électronique contenant au moins un composé selon une ou plusieurs des revendications 1 à 9.

14. Dispositif électronique selon la revendication 13, qui consiste en un dispositif électroluminescent organique, **caractérisé en ce que** le composé selon une ou plusieurs des revendications 1 à 9 est utilisé dans une couche d'émission en tant que matériau de matrice pour des émetteurs phosphorescents ou dans une couche de transport d'électrons ou dans une couche de transport de trous ou dans une couche de blocage d'excitons ou dans une couche de blocage de trous.
